# EUROPEAN PATENT SPECIFICATION

(11) **EP 2 230 311 B1**
(45) Date of publication and mention of the grant of the patent: **26.02.2014**
(21) Application number: 10161455.0
(22) Date of filing: 27.06.2006
(51) Int. Cl.: C12N 15/82, C12N 5/00, C12N 5/02

(54) **Components of cell culture media produced from plant cells**
Komponenten von aus Pflanzenzellen hergestellten Zellkulturmedien
Composants de milieux de culture de cellules produits à partir de cellules végétales

(30) Priority: 28.06.2005 US 694236 P
(43) Date of publication of application: 22.09.2010
(62) Divisional of application: 06774198.3
(73) Proprietor: Ventria Bioscience, Fort Collins, CO 80524 (US)
(72) Inventor: Deeter, Scott, Fort Collins Colorado 80525 (US); Schmidt, Joseph E., El Macero California 95618 (US); Huang, Ning, Davis California 95618 (US); Mabery, Kenneth J., Antelope California 95843 (US); Bethell, Delia R., West Sacramento California 95605 (US)
(74) Representative: Müller-Boré & Partner Patentanwälte PartG mbB

(56) References cited:
- EP-A1- 0 947 581
- WO-A-00/03012
- WO-A1-98/36085
- WO-A1-2005/011367
- WO-A2-01/72959
- US-A1- 2003 221 223
- DANIELL H ET AL: "Chloroplast-derived vaccine antigens and other therapeutic proteins" VACCINE, ELSEVIER LTD, GB LNKD- DOI:10.1016/J.VACCINE.2004.11.004, vol. 23, no. 15, 7 March 2005 (2005-03-07) , pages 1779-1783, XP004768053 ISSN: 0264-410X
- SIJMONS P C ET AL: "PRODUCTION OF CORRECTLY PROCESSED HUMAN SERUM ALBUMIN IN TRANSGENIC PLANTS" BIO/TECHNOLOGY, NATURE PUBLISHING CO. NEW YORK, US LNKD- DOI:10.1038/NBT0390-217, vol. 8, 1 March 1990 (1990-03-01), pages 217-221, XP000872477 ISSN: 0733-222X
- FERNÁNDEZ-SAN MILLÁN ALICIA ET AL: "A chloroplast transgenic approach to hyper-express and purify Human Serum Albumin, a protein highly susceptible to proteolytic degradation." PLANT BIOTECHNOLOGY JOURNAL MAR 2003 LNKD- PUBMED:17147744, vol. 1, no. 2, March 2003 (2003-03), pages 71-79, XP002595808 ISSN: 1467-7652
- WARTMANN THOMAS ET AL: "The ALEU2 gene: A new component for an Arxula adeninivorans-based expression platform" FEMS YEAST RESEARCH, WILEY-BLACKWELL PUBLISHING LTD, GB, NL LNKD- DOI:10.1016/S1567-1356(02)00190-3, vol. 3, no. 2, 1 March 2003 (2003-03-01), pages 223-232, XP002428713 ISSN: 1567-1356
- OKABAYSHI K ET AL: "SECRETORY EXPRESSION OF THE HUMAN SERUM ALBUMIN GENE IN THE YEAST, SACCHAROMYCES CEREVISIAE" JOURNAL OF BIOCHEMISTRY, JAPANESE BIOCHEMICAL SOCIETY / OUP, TOKYO; JP, vol. 110, no. 1, 1 January 1991 (1991-01-01), pages 103-110, XP000610608 ISSN: 0021-924X
- BELGACEM ET AL: "Isolation of esterified fatty acids bound to serum albumin purified from human plasma and characterised by MALDI mass spectrometry", BIOLOGICALS, ACADEMIC PRESS LTD., LONDON, GB, vol. 35, no. 1, 23 February 2007 (2007-02-23), pages 43-49, XP005899554, ISSN: 1045-1056, DOI: 10.1016/J.BIOLOGICALS.2006.01.004
- FRAHM GRANT E ET AL: "Investigation of the differences in thermal stability of two recombinant human serum albumins with 1,2-dipalmitoyl-sn-glycero-3-phosphocholin e liposomes by UV circular dichroism spectropolarimetry.", THE JOURNAL OF PHYSICAL CHEMISTRY. B 19 APR 2012, vol. 116, no. 15, 19 April 2012 (2012-04-19), pages 4661-4670, ISSN: 1520-5207
- HE YANG ET AL: "Large-scale production of functional human serum albumin from transgenic rice seeds.", PROCEEDINGS OF THE NATIONAL ACADEMY OF SCIENCES OF THE UNITED STATES OF AMERICA 22 NOV 2011, vol. 108, no. 47, 22 November 2011 (2011-11-22), pages 19078-19083, ISSN: 1091-6490
- KEENAN J ET AL: "Recombinant Human Albumin in Cell Culture: Evaluation of Growth-Promoting Potential for NRK and SCC-9 Cells In Vitro.", CYTOTECHNOLOGY SEP 1997, vol. 24, no. 3, September 1997 (1997-09), pages 243-252, ISSN: 0920-9069
- OHTANI W ET AL: "Physicochemical and immunochemical properties of recombinant human serum albumin from Pichia pastoris.", ANALYTICAL BIOCHEMISTRY 1 FEB 1998, vol. 256, no. 1, 1 February 1998 (1998-02-01), pages 56-62, ISSN: 0003-2697

## Description

This application claims priority benefit to U.S. provisional application Serial No. 60/694,236 filed June 28, 2006.

The present invention relates to a cell culture supplement as defined in claim 1.

Cell culture techniques allow animal or plant cells that are removed from tissues to grow when supplied with the appropriate nutrients and conditions. The cells are capable of dividing and can continue to grow until limited by some culture variables such as nutrient depletion or toxic buildup (Butler, M. & Jenkins, H., Nutritional aspects of growth of animal cells in culture, J of Biotechnol. (1989) 12: 97-110). Cell culture techniques have a number of applications including investigation of the normal physiology or biochemistry of cells (Balaban, B. & Urman, B., Embryo culture as a diagnostic tool, Reprod. Biomed. Online (2003) 7(6): 671-82), testing the effect of various chemical compounds or drugs on specific cell types (Farkas, D. & Tannenbaum, S.R., In vitro methods to study chemically-induced hepatotoxicity: a literature review, Curr. Drug Metab. (2005) 6(2): 111-25), studying the sequential or parallel combination of various cell types to generate artificial tissues (Wang et al., Cartilage tissue engineering with silk scaffolds and human articular chondrocytes, Biomaterials (2006)), and synthesizing valuable biologics from large scale cell cultures (Zeilinger et al., Three-dimensional co-culture of primary human liver cells in bioreactors for in vitro drug studies: effects of the initial cell quality on the long-term maintenance of hepatocytespecific functions, Altern. Lab Anim. (2002) 30(5): 525-38). Cell culture techniques have also been used for in vitro fertilization (Blake et al., Protein supplementation of human IVF culture media, J Assist. Reprod. Genet. (2002) 19(3): 137-43; Bungum et al., Recombinant human albumin as protein source in culture media used for IVF: a prospective randomized study, Reprod. Biomed. Online (2002) 4(3): 233-6), stem cell research (Conley et al., Derivation, propagation and differentiation of human embryonic stem cells, Int. J Biochem. Cell Biol. (2004) 36(4): 555-67), vaccine production (Chuang et al., Pharmaceutical strategies utilizing recombinant human serum albumin, Pharm. Res. (2002) 19(5): 569-77; GlaxoSmithKline, Publisher. HAVRIX® (Hepatitis A Vaccine, Inactivated) - Prescribing Information (2005), available at http://us.gsk.com/products/assets/us_havrix.pdf; Innis et al., Protection against hepatitis A by an inactivated vaccine, JAMA (1994) 271 (17): 1328-34; Merck, Publisher. PROQUAD® - Measles, Mumps, Rubella, and Varicella (Oka/Merck) Virus Vaccine Live - Prescribing Information (2005), available at http://www.merck.com/product/usa/pl_circulars/p/proquad/proquad_pl.pdf; Litwin, J., The growth of Vero cells in suspension as cell-aggregates in serum-free media, Cytotechnology (1992) 10(2): 169-74), tissue engineering including artificial skin (Atala, A., Future perspectives in reconstructive surgery using tissue engineering, Urol. Clin. North Am. (1999) 26(1): 157-65, ix-x; Sher, et al., Targeting perlecan in human keratinocytes reveals novel roles for perlecan in epidermal formation, J Biol. Chem. (2006) 281(8): 5178-87) and organs (Neronov et al., Integrity of endothelium in cryopreserved human cornea, Cryo Letters (2005) 26(2): 131-6; Han, et al., Interleukin-1alpha-induced proteolytic activation of metalloproteinase-9 by human skin, Surgery (2005) 138(5): 932-9) and gene and cell therapy (Chadd, H.E. & Chamow, S.M., Therapeutic antibody expression technology, Curr. Opin. Biotechnol. (2001) 12(2): 188-94).

The biologics encompass a broad range of cell products and include specific proteins or viruses that require animal cells for propagation. For example, therapeutic proteins such as monoclonal antibodies can be synthesized in large quantities by growing genetically engineered cells in large-scale cultures (Dewar et al., Industrial implementation of in vitro production of monoclonal antibodies, Ilar J (2005) 46(3): 307-13). The number of such commercially valuable biologics has increased rapidly over the last decade and has led to the present widespread interest in mammalian cell culture technology (Mizrahi, A., Biologicals produced from animal cells in culture-an overview, Biotechnol. Adv. (1988) 6(2): 207-20).

The major advantage of using cell culture for any of the above applications is the consistency and reproducibility of results that can be obtained from using a batch of clonal cells. The need for cell culture, especially at large scale, became apparent with the need for viral vaccines. Major epidemics of polio in the 1940s and 1950s promoted a lot of effort to develop an effective vaccine. In 1949, it was shown that poliovirus could be grown in cultures of human cells, which led to considerable interest in the development of large quantities of the polio vaccine using cell culture (Ligon, B.L., Thomas Huckle Weller MD: Nobel Laureate and research pioneer in poliomyelitis, varicella-zoster virus, cytomegalovirus, rubella, and other infectious diseases, Semin. Pediatr. Infect. Dis. (2002) 13(1): 55-63). The polio vaccine, produced from deactivated virus, became one of the first commercial products of cultured animal cells (Furesz, J., Developments in the production and quality control of poliovirus vaccines - Historical perspectives, Biologicals (2006)).

Many components of cell culture media are obtained from animal sources, including lactoferrin, transferrin and serum albumin. These components are extracted from animal sources, purified, and then combined with other inorganic ingredients to constitute cell culture media (Mizrahi, A. & Lazar, A., Media for cultivation of animal cells: an overview, Cytotechnology (1988) 1: 199-214). Cell culture components produced in this way have several undesirable features. For one, there is no way to ensure that extraneous animal products are completely excluded from the component, no matter how precise the purification techniques. Animal-derived components can carry the risk of contamination of pathogens of animal and human origin such as BSE, HIV and hepatitis virus (Chamberland et al., Emerging infectious disease issues in blood safety, Emerg. Infect. Dis. (2001) 7(3 Suppl): 552-3; Hepatitis C: A Brief Review, America's Blood Centers (October 1998) vol. 1, issue 3 (Strong, M. ed.), available at http://www.americasblood.org/index.cfm?fuseaction=display.showPage&pageID=133; Weinberg et al., Legal, financial, and public health consequences of HIV contamination of blood and blood products in the 1980s and 1990s, Ann. Intern. Med. (2002) 136(4): 312-9).

For another, small proteinaceous particles, such as prions, could be present and current scientific evidence suggests that even a very small number could result in disease. In a recent case, a healthy subject died from possible transmission of variant Creutzfeldt-Jakob disease from blood transfusion (Llewelyn et al., Possible transmission of variant Creutzfeldt-Jakob disease by blood transfusion, Lancet (2004) 363(9407): 417-21). This raises concerns regarding the consistent safety of blood and blood-derived products (Bird, S.M., Recipients of blood or blood products "at vCJD risk," BMJ (2004) 328(7432): 118-9). Because of such safety concerns, regulators are encouraging replacing animal components with recombinant proteins in vaccine production and other applications of cell culture techniques (Egan, W.M., Bovine-derived products used in the manufacture and formulation of vaccines: current policies and issues for the future (2004), available at http://www.fda.gov/cber/summaries/pda092004we.pdf; European Medicine Agency (EMA), CPMP position statement on Crutzfeldt-Jakob disease and plasma-derived and urine-derived medicinal products (2003), available at http://www.emea.eu.int/pdfs/human/press/pos/287902en.pdf; World Health Organization (WHO), Variant Creutzfeldt-Jakob disease (2002), available at http://www.who.int/mediacentre/factsheets/fs180/en/). Further, many users may be hesitant to use products from cell culture media that contain animal components for religious or moral reasons (Sarkar, S., Use of animal products in vegetarians and others, Anaesthesia (2005) 60(5): 519-20).

Therefore, there is a need to produce components for incorporation into cell culture media that is not derived from, or produced by, animal sources, yet still retain their functional value for cell cultures.

Accordingly, it is an objective of the invention to provide cell culture media supplement, comprising at least one plant-produced heterologous protein as a cell culture media component, wherein the protein is recombinant human serum albumin produced in a rice plant, that improves growth and productivity of cells compared to the use of media containing animal-derived human serum albumin when cultivating AE1 hybridoma cells, wherein the cell culture media supplement still contains other components from the purifying or isolating step(s) of the human serum albumin.

Another embodiment of the invention comprises a cell culture media comprising the cell culture supplement of the present invention. For example, in the cell culture media supplement of the present invention, the human serum albumin is produced by the following process:
a) transforming a rice cell with a chimeric gene comprising:
   (i) a promoter from the gene of a seed storage protein;
   (ii) a first DNA sequence, operably linked to the promoter, encoding a seed storage protein, and
   (iii) a second DNA sequence, operably linked to the promoter, encoding the heterologous protein, wherein the first and second DNA sequences are linked in translation frame and together encode a fusion protein comprising the storage protein and the heterologous protein; and
b) growing a plant from the transformed plant cell for a time sufficient to produce seeds containing the heterologous protein. The embodiment may optionally include purifying the heterologous protein from the harvested seeds.

A method for producing a cell culture media supplement may be a method comprising:
a) transforming a plant cell with a chimeric gene comprising:
   (i) a promoter from the gene of a seed storage protein;
   (ii) a first DNA sequence, operably linked to the promoter, encoding a seed storage protein, and
   (iii) a second DNA sequence, operably linked to the promoter, encoding the heterologous protein, wherein the first and second DNA sequences are linked in translation frame and together encode a fusion protein comprising the storage protein and the heterologous protein;
b) growing a plant from the transformed plant cell for a time sufficient to produce seeds containing the heterologous protein;
c) harvesting the seeds from the plant; and
d) combining the heterologous protein with a cell culture media.

Another example for producing the cell culture media supplement of the present invention comprises a method for creating a recombinant rice cell that can produce components that may be incorporated into cell culture media or supplement for cell culture media.

Another example for producing the cell culture media supplement of the present invention comprises a method of transforming a rice cell by incorporating a DNA segment that encodes for a component of cell culture media.

Another example for producing the cell culture media supplement of the present invention comprises a method of transforming a rice cell by incorporating a DNA segment that encodes for components of cell culture media such as amino acids, growth factors, and other cell culture proteins including lactoferrin and human serum albumin.

The cell culture supplement according to the present invention can be used for a cell culture medium with components that are derived from plants, and more preferably all the organic ingredients are derived exclusively from non-animal sources.

The cell culture supplement according to the present invention can be used for a method for the production of cell culture media, the method comprising incorporating into cell culture media components derived from recombinant rice cells.

Another aspect of the invention comprises of the use of the media supplemented according to the present invention to improve cell growth and productivity in cell cultures.

The cell culture supplement according to the present invention can be used for an improved cell culture media comprising one or more media ingredients and as a supplement to one or more cell culture media components derived from rice cells.

The cell culture supplement according to the present invention can be used for a method for achieving high growth rate of culture cells and high productivity of culture cells by culturing the culture cell in the improved cell culture media.

Another example for producing the cell culture media supplement of the present invention comprises a method of producing an improved cell culture media comprising a) obtaining a rice cell transformed with a vector containing a plant-based promoter and a gene encoding a cell culture media component, b) cultivating the transformed plant cells to set seeds, c) harvesting the mature seeds, d) extracting and purifying of the cell culture components from the seed, and e) adding the components to cell culture media.

Figure 1 is a comparison of the codon-optimized epidermal growth factor sequence ("Egfactor") with a native epidermal growth factor sequence ("Native Gene"), aligned to show 53 codons in the mature sequences, with 27 (51%) codon changes and 30 (19%) nucleotide changes.

Figure 2 is a restriction map of the 3,877 bp plasmid, API303 (pGt1-EGF v2.1), showing an expression cassette for epidermal growth factor (EGF), and containing a rice Gt1 promoter, a Gt1 signal peptide, codon optimized EGF, a Nos terminator and an ampicillin resistance selectable marker.

Figure 3 is a restriction map of the 4,142 bp plasmid, API270 (pGlb-EGF v2.1), showing an expression cassette for epidermal growth factor ("EGF"), and containing a Glb promoter, a Glb signal peptide, codon optimized EGF, a Nos terminator and an ampicillin resistance selectable marker.

Figure 4 is a Western blot analysis of recombinant human EGF ("rhEGF") in the R1 generation of transgenic rice seeds. Lane 1 indicates extracts from seeds of control untransformed Taipei 309 rice variety (Oryza sativa, Japonica). Lanes 2 to 5 show rhEGF expressed in the seed extracts obtained from independent transgenic rice events. Lane 6 shows a purified rhEGF standard expressed in yeast, loaded at 125 ng. Lane 7 shows a broad range of molecular weight markers.

Figure 5 is a comparison of the codon-optimized insulin-like growth factor I sequence ("Insgfact") with a native human insulin-like growth factor I sequence ("native gene"), aligned to show 70 codons in the mature sequences, with 40 (57%) codon changes and 47 (22%) nucleotides changes.

Figure 6 is a restriction map of the 4,194 bp plasmid, API271 (pGlb-IGF v2.1), showing an expression cassette for insulin-like growth factor I ("IGF"), and containing a Glb promoter, a Glb signal peptide, codon optimized IGF, a Nos terminator and an ampicillin resistance selectable marker.

Figure 7 is a restriction map of the 3,928 bp plasmid, API304 (pGt1-IFG v2.1), showing an expression cassette for insulin-like growth factor I ("IGF"), and containing a rice Gt1 promoter, a Gt1 signal peptide, codon optimized IGF, a Nos terminator and an ampicillin resistance selectable marker.

Figure 8 is a Western blot analysis of recombinant human IGF-I ("rhIGF") expressed in the R1 generation of transgenic rice seeds. Lane 1 shows rice seed extract from seeds of control untransformed rice variety Taipei 309. Lanes 2 to 8 show rhIGF expressed in seed extracts obtained from seven independent transgenic rice events. Lane 9 shows a purified rhIGF-1 standard expressed in yeast, loaded at 1 µg. Lane 10 shows a broad range of molecular weight markers.

Figure 9 is a comparison of the codon-optimized intestinal trefoil factor sequence ("Trefoil") with a native intestinal trefoil factor sequence ("Native Gene"), aligned to show 60 codons in the mature sequences, with 26 (43%) codon changes and 28 (15%) nucleotide changes.

Figure 10 is a restriction map of the 4,163 bp plasmid, API269 (pGlb-ITF-nos), showing an expression cassette for intestinal trefoil factor ("ITF"), and containing a Glb promoter, a Glb signal peptide, codon optimized ITF, a Nos terminator and an ampicillin resistance selectable marker.

Figure 11 is a restriction map of the 3,889 bp plasmid, API307 (pGt1-ITF-nos), showing an expression cassette for intestinal trefoil factor (ITF), and containing a rice Gt1 promoter, a Gt1 signal peptide, codon optimized ITF, a Nos terminator and an ampicillin resistance selectable marker.

Figure 12 is a Western blot analysis of recombinant human ITF ("rhITF") expression in the R1 generation of transgenic rice seeds. Lane 1 indicates extracts from seeds of control untransformed Taipei 309 rice variety. Lanes 2 and 3 show rhITF expressed in the seed extracts obtained from two independent transgenic rice events. Lane 4 indicates a purified rhITF standard expressed in yeast, loaded at 1 µg. Lane 5 shows a broad range of molecular weight markers.

Figure 13 is a comparison of the codon-optimized human lactoferrin sequence ("cod opt lac") with a native human lactoferrin sequence ("native lac"), aligned to show 693 codons, with 413 (59.6%) codon changes and 467 (22.5%) nucleotide changes.

Figure 14 is a plasmid map of the 5,817 bp plasmid, API164 (GT1-Lac), showing an expression cassette for human lactoferrin and containing a Gt1 promoter, a Gt1 signal peptide, codon optimized human lactoferrin, a Nos terminator and a kanamycin resistance selectable marker.

Figure 15 shows the results of a SDS-PAGE analysis for the expression of recombinant human lactoferrin. Total proteins from rice seed extracts were suspended in Laemli sample buffer, run on a gradient gel and stained with Coomassie blue. Lane 1 is the molecular weight marker. Lanes 3 to 6 are purified human derived lactoferrin (Sigma Chemical, USA). Lanes 8 to 13 are single seed extracts from homozygous independent transgenic rice lines and lane 14 is a seed extract from non-transformed rice variety Taipei 309.

Figure 16 is a stable expression of recombinant human lactoferrin in transgenic rice grains from R₂ through R₁₀ generations. Total soluble proteins from 1 g of brown rice flour was extracted with 40 ml of extraction buffer and clarified by centrifugation. The extract was analyzed via ELISA. Extraction was repeated three times and standard deviation is shown as an error bar.

Figure 17 shows DNA and protein sequence of fusion between Gt1 signal peptide (Gt1 SP) and codon-optimized human serum albumin (OPTIMIZED HSA) sequence based on native protein sequence derived from P02768 (Swiss-Prot).

Figure 18 is a plasmid map of the 5,496 bp plasmid, API504 (GT1-HSA), showing an expression cassette for human serum albumin and containing a Gt1 promoter, a Gt1 signal peptide, codon optimized human serum albumin, a Nos terminator and a kanamycin resistance selectable marker.

Figure 19 is a plasmid map of the 12,388 bp plasmid, API508 (JH/GT1-HSA), showing an expression cassette for human serum albumin and containing a Gt1 promoter, a Gt1 signal peptide, codon optimized human serum albumin, a Nos terminator and a kanamycin resistance selectable marker.

Figure 20 shows the effect of three forms of recombinant human lactoferrin on cell growth. The three forms of lactoferrin are asis-lactoferrin, apo-lactoferrin and holo-lactoferrin. Asis-lactoferrin is the same as partial-lactoferrin with approximately 50% iron saturation. The baseline is with basal media and the rest is baseline supplemented with various concentration of recombinant human lactoferrin as indicated in the graph. Cell growth is measured as thymidine incorporation.

Figure 21 shows the effect of three forms of recombinant human lactoferrin on cell growth. The three forms of lactoferrin are asis-lactoferrin, apo-lactoferrin and holo-lactoferrin. Asis-lactoferrin is the same as partial-lactoferrin with approximate 50% iron saturation. The baseline is with basal media plus 5% fetal calf serum and the rest are baseline supplemented with epidermal growth factor, 5% FCS or recombinant human lactoferrin at a final concentration of 1 mg/ml. Cell growth is measured as number of cells/ml.

Figure 22 shows cell culture media supplemented with recombinant human lactoferrin (LACROMIN^{™}) expressed in rice grain promotes hybridoma cell growth and increases hybridoma cell number as compared to animal derived transferrin. FBS is fetal bovine serum. AFM6 is serum-free cell culture media derived from KC Bio, Kansas. AFM6-Fe is a serum-free media without iron.

Figure 23 shows the effect of recombinant human serum albumin (rHSA) and plasma-derived human serum albumin (pHSA) on the growth of hybridoma AE1 cells in control medium (CM) containing reduced serum (1% FBS).

Figure 24 shows the effect of recombinant human serum albumin (rHSA) and plasma-derived human serum albumin (pHSA) on the growth of hybridoma AE1 cells in serum-free medium (SMF).

Unless otherwise indicated, all terms used herein have the meanings given below or are generally consistent with same meaning that the terms have to those skilled in the art of the present invention. Practitioners are particularly directed to Sambrook et al. (1989) Molecular Cloning: A Laboratory Manual (Second Edition), Cold Spring Harbor Press, Plainview, N.Y., Ausubel FM et al. (1993) Current Protocols in Molecular Biology, John Wiley & Sons, New York, N.Y., and Gelvin and Schilperoot, eds. (1997) Plant Molecular Biology Manual, Kluwer Academic Publishers, The Netherlands for definitions and terms of the art.

General and specific techniques for producing proteins from plant cells may be obtained from the following patents and applications, each of which is incorporated herein in its entirety by reference: U.S. Pat. Appl. Pub. No. 2003/0172403 A1 ("Plant Transcription Factors and Enhanced Gene Expression"); U.S. Pat. No. 6,991,824 ("Expression of Human Milk Proteins in Transgenic Plants"); U.S. Pat. Appl. Pub. No. 2003/0221223 ("Human Blood Proteins Expressed in Monocot Seeds"); U.S. Pat. Appl. Pub. No. 2004-0078851 ("Production of Human Growth Factors in Monocot Seeds"); U.S. Pat. Appl. Pub. No. 2004/0063617 ("Method of Making an Anti-infective Composition for Treating Oral Infections"); and international application no. PCT/US2004/041083 ("High-level Expression of Fusion Polypeptides in Plant Seeds Utilizing Seed-Storage Proteins as Fusion Carriers"). Other general and specific techniques for producing proteins from plant cells may be obtained, for example, from the following references: U.S. Patent No. 5,693,507, U.S. Patent No. 5,932,479, U.S. Patent No. 6,642,053, and 6,680,426 (each titled "Genetic Engineering of Plant Chloroplasts"); U.S. Pat. Appl. Pub. No. 2005/0066384 ("Site-Targeted Transformation Using Amplification Vectors"); U.S. Pat. Appl. Pub. No. 2005/0221323 ("Amplification Vectors Based on Trans-Splicing"); U.S. Pat. Appl. Pub. No. 2006/0026718 ("Method of Controlling Cellular Processes in Plants"); and U.S. Pat. Appl. Pub. No. 2006/0075524 (Method of Controlling A Cellular Process in a Multi-Cellular Organism"); Marillonnet et al., Systemic Agrobacterium tumefaciens-mediated transfection of viral replicons for efficient transient expression in plants, Nature Biotech. (2005) 23(6): 718-723.

The polynucleotides as defined herein may be in the form of RNA or in the form of DNA, and include messenger RNA, synthetic RNA and DNA, cDNA, and genomic DNA. The DNA may be double-stranded or single-stranded, and if single-stranded may be the coding strand or the non-coding (anti-sense, complementary) strand.

The term "stably transformed" with reference to a plant cell means the plant cell has a non-native (heterologous) nucleic acid sequence integrated into its genome which is maintained through two or more generations.

By "host cell" is meant a cell containing a vector and supporting the replication and/or transcription and/or expression of the heterologous nucleic acid sequence. Preferably, according to the invention, the host cell is a plant cell. Other host cells may be used as secondary hosts, including bacterial, yeast, insect, amphibian or mammalian cells, to move DNA to a desired plant host cell.

A "plant cell" refers to any cell derived from a plant, including undifferentiated tissue (e.g., callus) as well as plant seeds, pollen, propagules, embryos, suspension cultures, meristematic regions, leaves, roots, shoots, gametophytes, sporophytes and microspores.

The term "mature plant" refers to a fully differentiated plant.

The term "seed product" includes; but is not limited to, seed fractions such as de-hulled whole seed, flour (seed that has been de-hulled by milling and ground into a powder) a seed extract, preferably a protein extract (where the protein fraction of the flour has been separated from the carbohydrate fraction), malt (including malt extract or malt syrup) and/or a purified protein fraction derived from the transgenic grain.

The term "biological activity" refers to any biological activity typically attributed to that protein by those skilled in the art.

"Monocot seed components" refers to carbohydrate, protein, and lipid components extractable from monocot seeds, typically mature monocot seeds.

"Seed maturation" refers to the period starting with fertilization in which metabolizable reserves, e.g., sugars, oligosaccharides, starch, phenolics, amino acids, and proteins, are deposited, with and without vacuole targeting, to various tissues in the seed (grain), e.g., endosperm, testa, aleurone layer, and scutellar epithelium, leading to grain enlargement, grain filling, and ending with grain desiccation.

"Maturation-specific protein promoter" refers to a promoter exhibiting substantially up-regulated activity (greater than 25%) during seed maturation.

The term "growth factor" refers to proteins, or biologically active fragments thereof, including, without limitation, epidermal growth factor (EGF), keratinocyte growth factors (KGF) including KGF-1 and KGF-2, insulin-like growth factors (IGF) including IGF-I and IGF-II, Intestinal trefoil factor (ITF), transforming growth factors (TGF) including TGF-α and -β1-3, granulocyte colony-stimulating factor (GCSF), nerve growth factor (NGF) including NGF-β, and fibroblast growth factor (FGF) including FGF-1-19 and -12β, and biologically active fragments of these proteins. The sequences of these and other growth factors are well-known to those of ordinary skill in the art.

"Heterologous DNA" refers to DNA which has been introduced into plant cells from another source, or which is from a plant source, including the same plant source, but which is under the control of a promoter that does not normally regulate expression of the heterologous DNA.

"Heterologous protein" is a protein encoded by a heterologous DNA. The proteins include, but are not limit to, growth factor(s), lactoferrin, transferrin, albumin, insulin, and fractions thereof, growth hormone and fractions thereof, Fibronectin (human) - attachment factor, Lamin (Mouse) - attachment factor, collagenase , platelet derived growth factor, Human brain-derived neurotrophic factor (BDNF), glial-derived neurotrophic factor (GDNF), thymic factors, haptocorin, lactahedrin, lactoperoxidase. Alpha-fetoprotein, immunoglobin, alpha-lactalbumin.

As used herein, the terms "native" or "wild-type" relative to a given cell, protein, polypeptide, nucleic acid, trait or phenotype, refers to the form in which that is typically found in nature.

As used herein, the term "purifying" is used interchangeably with the term "isolating" and generally refers to any separation of a particular component from other components of the environment in which it is found or produced. For example, purifying a recombinant protein from plant cells in which it was produced typically means subjecting transgenic protein-containing plant material to separation techniques such as sedimentation, centrifugation, filtration, and chromatography. The results of any such purifying or isolating step(s) may still contain other components as long as the results enrich for the component of interest.

As used herein, the terms "transformed" or "transgenic" with reference to a host cell means the host cell contains a non-native or heterologous or introduced nucleic acid sequence that is absent from the native host cell. Further, "stably transformed" in the context of the present invention means that the introduced nucleic acid sequence is maintained through two or more generations of the host, which is preferably (but not necessarily) due to integration of the introduced sequence into the host genome.

"Cell culture media" refers to media for cell culture purpose which includes complete media, basal media or basal media supplemented with cell culture media component, cell culture media supplement, or media with various amounts of serum or chemically defined media.

"Cell culture media component" refers any heterologous proteins for use as a supplement to cell culture media.

"Cell culture media ingredient" includes cell culture media components, proteins, peptides, hormones, carbohydrates, amino acids, lipids, vitamins, antibiotics, organic and inorganic salts.

"Cell culture media supplement" refers to a combination of one or multiple cell culture media components with or without other ingredients for addition to cell culture media.

Most cells in culture grow best at 37°C and at pH 7.4 with appropriate media. Typically, cell culture media contains carbohydrates, amino acids, various salts, bicarbonate to maintain pH, vitamins and hormones, and phenol red as pH indicator. Examples of cell culture media that can be formed include: Dulbecco's Modified Eagle's Medium (DME), Ham's Nutrient Mixtures, MCDB Media, Minimum Essential Medium Eagle, RPMI Media, Ames' Media, BGJb Medium (Fitton-Jackson Modification), Click's Medium, CMRL-1066 Medium, Fischer's Medium, Glascow Minimum Essential Medium (GMEM), Iscove's Modified Dulbecco's Medium (IMDM), L-15 Medium (Leibovitz), McCoy's 5A Modified Medium, NCTC Medium, Swim's S-77 Medium, Waymouth Medium, and William's Medium E.

Frequently, cell culture media is supplemented with serum at concentration of 5 to 10%. Serum is a complex mixture containing undefined essential matter for cell growth. Commonly used serum is fetal calf serum (FCS) and fetal bovine serum (FBS). Due to its nature of complex mixture, lack of consistency and potential risk of pathogen contamination, cell culture industries and regulatory agencies are seeking alternatives to adding serum to cell culture media.

Since cells grow poorly in basal media, the basal media is then supplemented with various growth factors, hormones, and other essential components to generate serum-free media but maintaining the same cell growth rate as in serum-based media. Human transferrin or bovine transferrin is added as a source of iron. Typically, serum-free media contains insulin, serum albumin, transferrin, insulin-like growth factor and epidermal growth factor. Other proteins such as lactoferrin have also been shown to be able to promote cell growth. Each component provides a distinct function in cell growth. In the art, these protein components are animal-derived. With the increase in the concern of potential pathogen contamination from animal sourced material, cell culture industries are trying to develop animal-component-free media. While some successes have been made with the use of plant-based hydrolysates or recombinant protein produced with microbial system, plant-based hydrolysate is an undefined mixture of components and recombinant protein from microbial sources are too expensive to use in routine cell culture. An alternative source of non-animal-derived components is urgently needed for cell culture industries.

The cell culture media components produced by the methods to be described in the following sections are entirely plant-based and may be combined with inorganic salts such as NaCl, KCI, NaH₂PO₄, NaHCO₃, CaCl₂, and MgCl₂ and other ingredients such as amino acids, vitamins, growth factors, sugars, and antibiotics to form a variety of different cell culture media.

Expression vectors for use in the present invention are chimeric nucleic acid constructs (or expression vectors or cassettes), designed for operation in plants, with associated upstream and downstream sequences.

In general, expression vectors for use in practicing the invention include the following operably linked elements that constitute a chimeric gene: a promoter derived from a gene encoding a plant protein, operatively linked to a gene encoding a heterologous protein. The vector can include a promoter from the gene of a maturation-specific monocot plant storage protein, a first DNA sequence, operably linked to the promoter, encoding a monocot plant seed-specific signal sequence (such as an N-terminal leader sequence or a C-terminal trailer sequence) capable of targeting a polypeptide linked thereto to an endosperm cell, preferably an endosperm-cell organelle, such as a protein storage body, and a second DNA sequence, linked in translation frame with the first DNA sequence, encoding a cell culture media component. The signal sequence is preferably cleaved from the cell culture media component in the plant cell.

The chimeric gene, in turn, is typically placed in a suitable planttransformation vector having (i) companion sequences upstream and/or downstream of the chimeric gene which are of plasmid or viral origin and provide necessary characteristics to the vector to permit the vector to move DNA from bacteria to the desired plant host; (ii) a selectable marker sequence; and (III) a transcriptional termination region generally at the opposite end of the vector from the transcription initiation regulatory region.

Numerous types of appropriate expression vectors, and suitable regulatory sequences are known in the art for a variety of plant host cells. The promoter region is chosen to be regulated in a manner allowing for induction under seed-maturation conditions. In one aspect of this embodiment of the invention, the expression construct includes a promoter which exhibits specifically up-regulated activity during seed maturation. Promoters for use in the invention are typically derived from cereals such as rice, barley, wheat, oat, rye, corn, millet, triticale or sorghum. Examples of such promoters include the maturation-specific promoter region associated with one of the following maturation-specific monocot plant storage proteins: rice glutelins, oryzins, and prolamines, barley hordeins, wheat gliadins and glutelins, maize zeins and glutelins, oat glutelins, and sorghum kafirins, millet pennisetins, and rye secalins.

One example of the present invention involves high-level expression of heterologous molecules that are components of cell culture media. These components can be expressed in a plant cell by a chimeric gene comprising the gene(s) encoding the desired component operably linked with an endogenous plant protein encoding gene. In a preferred embodiment the endogenous plant encoding gene is a rice seed storage protein gene for expression in a rice mature seed expression system.

The expressed polypeptide may be a multi-domain polypeptide with one domain being the heterologous or exogenous cell culture component and the other being the endogenous plant protein. At least one selective purification tag and/or at least one specific protease cleavage site may be provided for eventual release of the cell culture media component from the monocot seed storage protein carrier. For example, a strategic methionine or tryptophan residue providing a chemical cleavage site may be engineered in frame between the domains for release of the cell culture component from the endogenous plant protein. This technique is useful for cell culture media components that have protein portions or are proteins, for example lactoferrin, human serum albumin, and human growth factors.

Other selective protease cleavage sites include, but are not limited to enterokinase (ek), Factor Xa, thrombin, V8 protease, GENENASE™(a variant of subtilisin BPN'), α-lytic protease or tobacco etch virus protease. Alternatively, cleavage of the fusion protein could be performed via chemical cleaving agents such as cyanogen bromide or N-chlorosuccinimide.

The invention provides cell culture media components recombinantly produced in host rice seed wherein the cell culture media component expressed comprises about 3% or greater of the total soluble protein in the monocot seed. Thus, for example, the yield of total soluble protein which comprises the cell culture media component targeted for production can be from about 3% to about 5%, from about 5% to about 10%, from about 10% to about 15%, from about 15% to about 20%, from about 20% to about 25%, and from about 25% to about 30% of the total soluble protein found in the recombinantly engineered production plant seed. Additionally, for example, the yield of total soluble protein which comprises the cell culture media component targeted for production can be about 3% or greater, about 5% or greater, about 10% or greater, about 15% or greater, about 20% or greater, and about 25% or greater.

In some embodiments, the cell culture media is a reduced serum or serum-free medium. In other embodiments, the cell culture media is disclosed as a complete media, basal media, or basal media supplemented with a cell culture media component.

The cell culture supplement according to the present invention can be used in an improved cell culture media having one or more proteinaceous cell culture media components, the improvement comprising at least one plant-produced heterologous protein as a cell culture media component, wherein the heterologous protein is produced by the following process:
a) transforming a plant cell with a chimeric gene comprising:
   (i) a promoter from the gene of a seed storage protein;
   (ii) a first DNA sequence, operably linked to the promoter, encoding a seed storage protein, and
   (iii) a second DNA sequence, operably linked to the promoter, encoding the heterologous protein, wherein the first and second DNA sequences are linked in translation frame and together encode a fusion protein comprising the storage protein and the heterologous protein; and
b) growing a plant from the transformed plant cell for a time sufficient to produce seeds containing the heterologous protein wherein the heterologous protein is human serum albumin and the plant is rice. In some embodiments, the heterologous protein is purified from the harvested seeds. In some embodiments, the heterologous protein constitutes at least about 3.0% of the total soluble protein in the harvested seeds.

For example there is a method for producing a cell culture media having at least one plant-produced heterologous protein as a cell culture media component, the method comprising:
a) transforming a plant cell with a chimeric gene comprising:
   (i) a promoter from the gene of a seed storage protein;
   (ii) a first DNA sequence, operably linked to the promoter, encoding a seed storage protein, and
   (iii) a second DNA sequence, operably linked to the promoter, encoding the heterologous protein, wherein the first and second DNA sequences are linked in translation frame and together encode a fusion protein comprising the storage protein and the heterologous protein;
b) growing a plant from the transformed plant cell for a time sufficient to produce seeds containing the heterologous protein;
c) harvesting the seeds from the plant; and
d) combining the heterologous protein with a cell culture media.

For example there is a method of producing monocot seeds, preferably rice grains, that can express the desired cell culture media component, comprising:
a) transforming a monocot plant cell with a chimeric gene comprising:
   (i) a promoter from the gene of a monocot seed storage protein;
   (ii) a first DNA sequence, operably linked to the promoter, encoding a monocot seed storage protein; and
   (iii) a second DNA sequence, operably linked to the promoter, encoding a cell culture media component,
   wherein the first and second DNA sequences are linked in translation frame and together encode a fusion protein comprising the storage protein and the cell culture media component;
b) growing a monocot plant from the transformed monocot plant cell for a time sufficient to produce seeds containing the cell culture media component; and
c) harvesting the seeds from the monocot plant.

### EXAMPLES

### Comparative Example 1: Expression of recombinant human growth factors

In general, expression vectors were constructed using standard molecular biological techniques. The vectors contain a heterologous protein coding sequence for certain growth factors under the control of a rice tissue-specific promoter, as further described below.

The nucleotide sequence of the promoter and the nucleotide sequence of the signal peptide of the rice glutelin-1 (Gt1) gene were cloned based on the published Gt1 gene sequence. The nucleotide sequence of the promoter and the nucleotide sequence of the signal peptide of the rice globulin (Glb) gene were cloned based on the published Glb gene sequence.

### A. Generation of human EGF expression vector

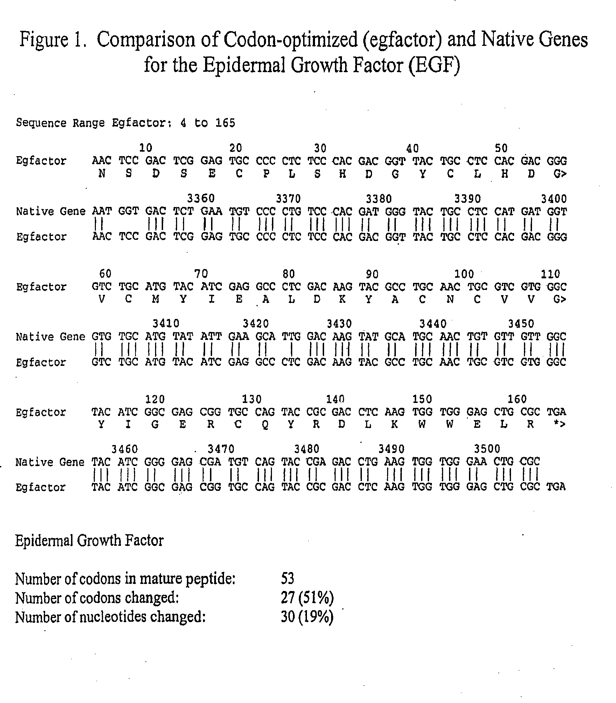

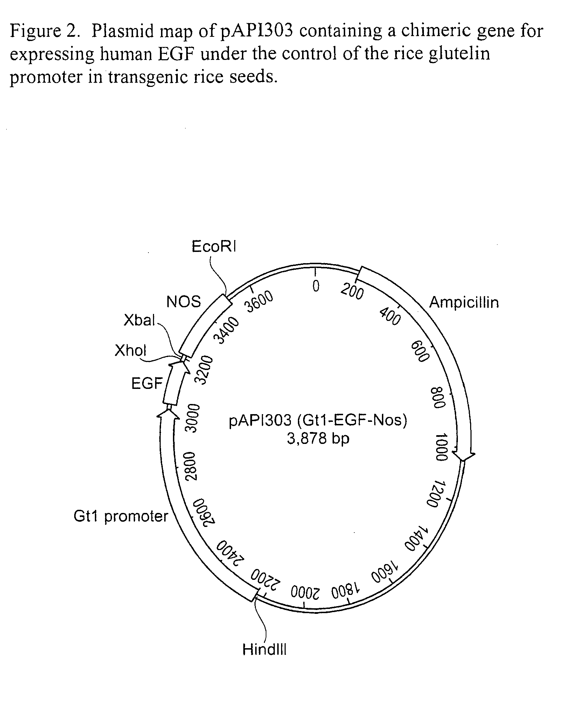

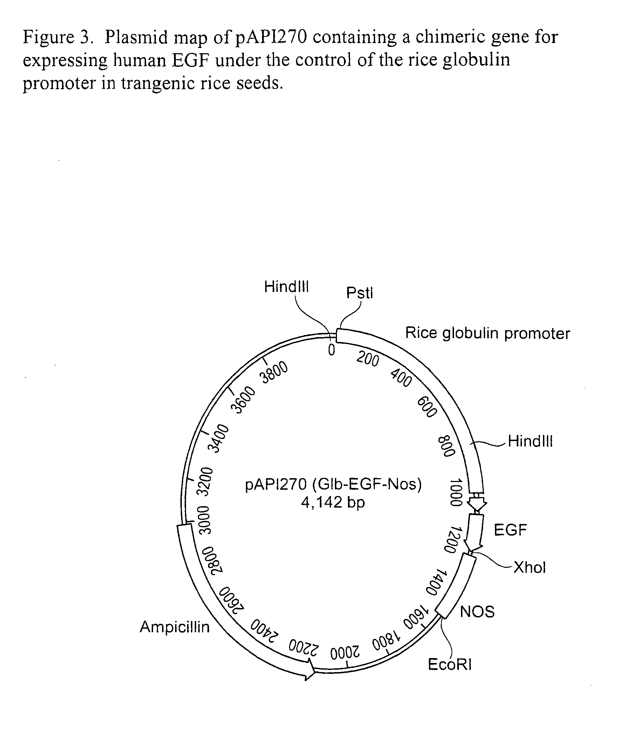

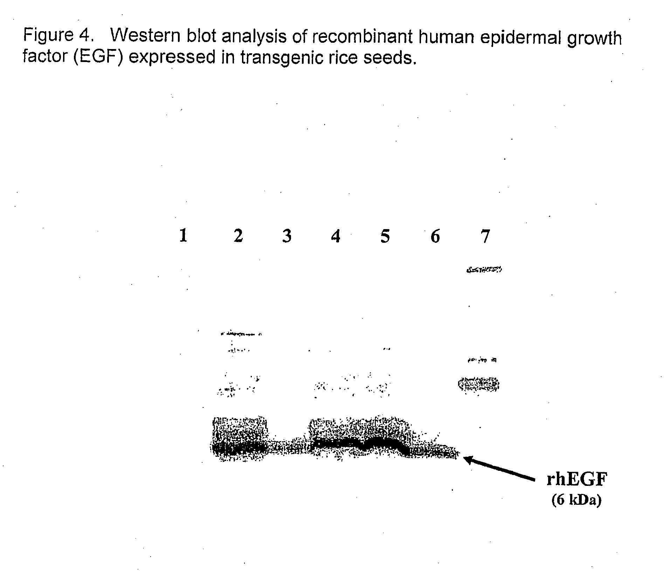

The human EGF gene was codon optimized as shown in Figure 1, and synthesized by Operon Technologies (CA, USA) (SEQ ID NO: 1). For expression of EGF in rice seeds, the codon optimized gene was operably linked to the rice endosperm specific glutelin (Gt1) promoter, Gt1 signal peptide and NOS terminator in pAPI303 (Figure 2), and to the rice endosperm specific globulin (Glb) promoter, Glb signal peptide and NOS terminator in pAPI270 (Figure 3). The transgenic plant expressing EGF was generated, and plant-generated recombinant EGF was detected, as shown in Figure 4 and as exemplified herein.

### B. Generation of human IGF-I expression vector

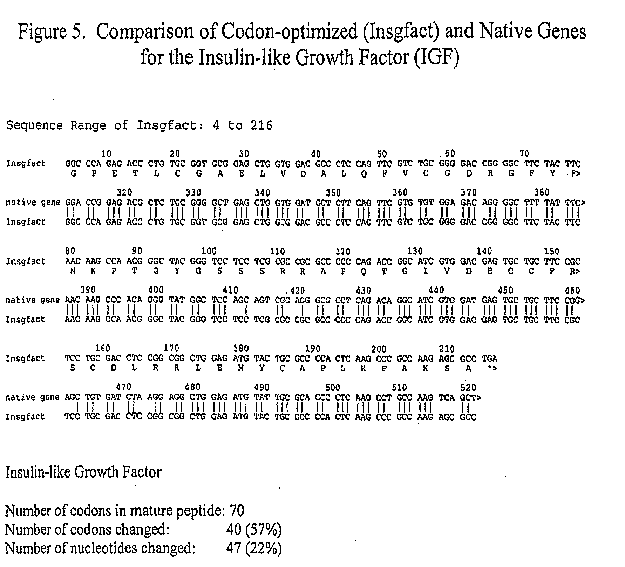

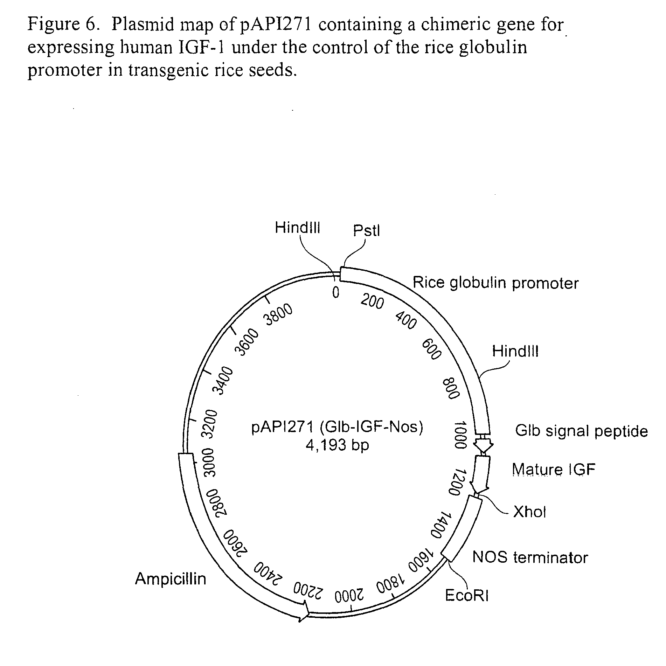

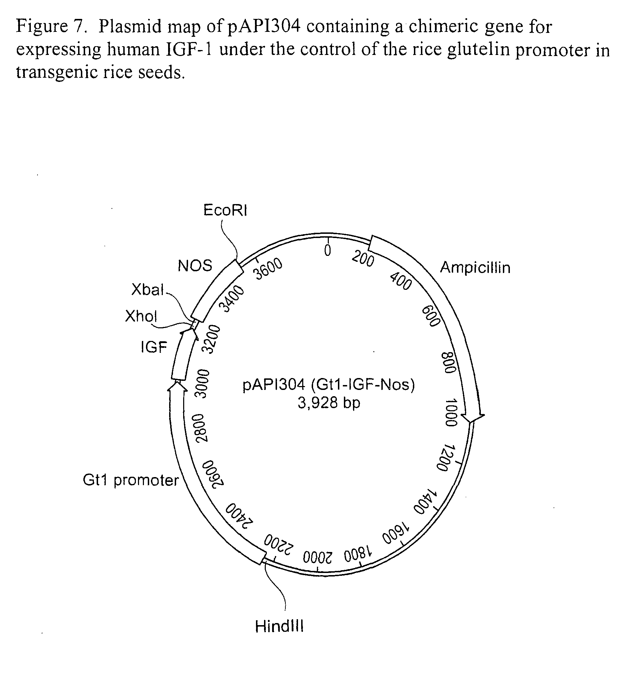

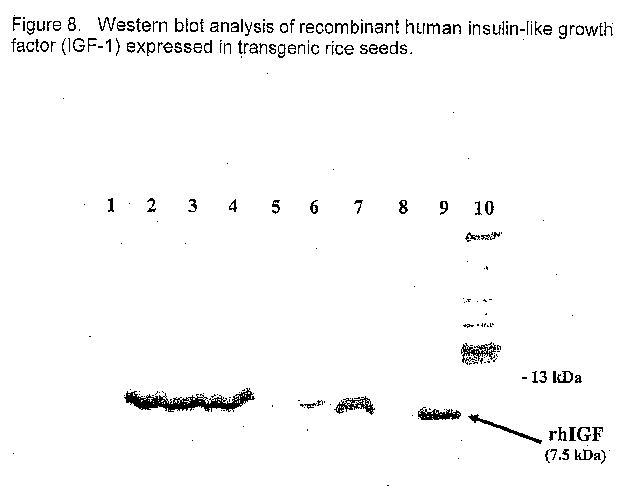

The IGF-I gene was codon optimized as shown in Figure 5, and synthesized by Operon Technologies (CA, USA) (SEQ ID NO: 3). For expression of IGF-I in rice seeds, the codon optimized gene was operably linked to the rice endosperm specific glutelin (Gt1) promoter, Gt1 signal peptide and NOS terminator in pAPI304 (Figure 7), and to the rice endosperm specific globulin (Glb) promoter, Glb signal peptide and NOS terminator in pAPI271 (Figure 6). The transgenic plant expressing IGF-I was regenerated, and plant-generated recombinant IGF-I was detected as shown in Figure 8 and as exemplified herein.

### C. Generation of human ITF

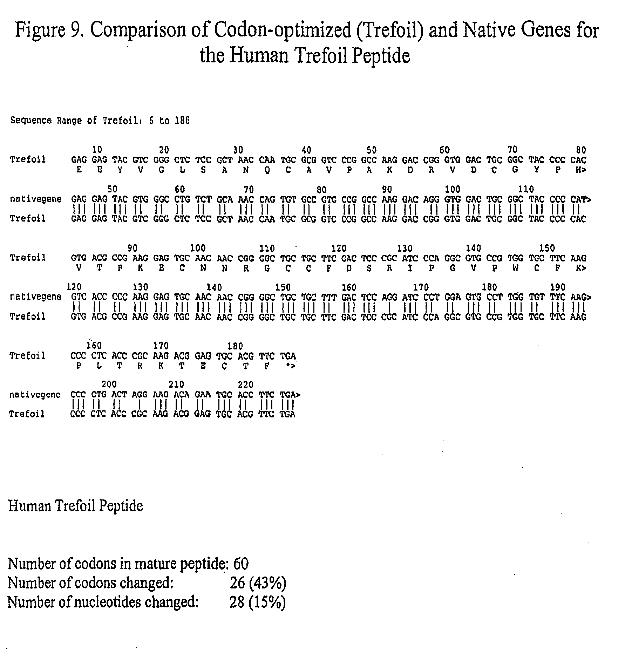

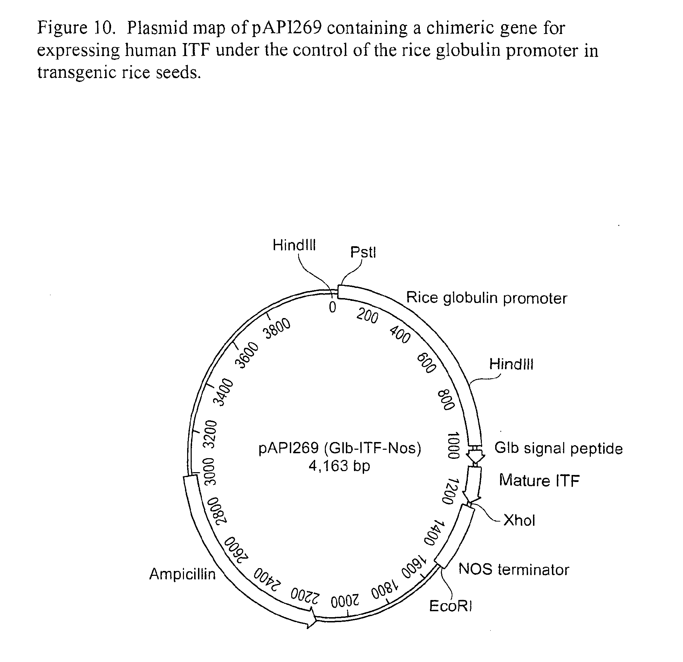

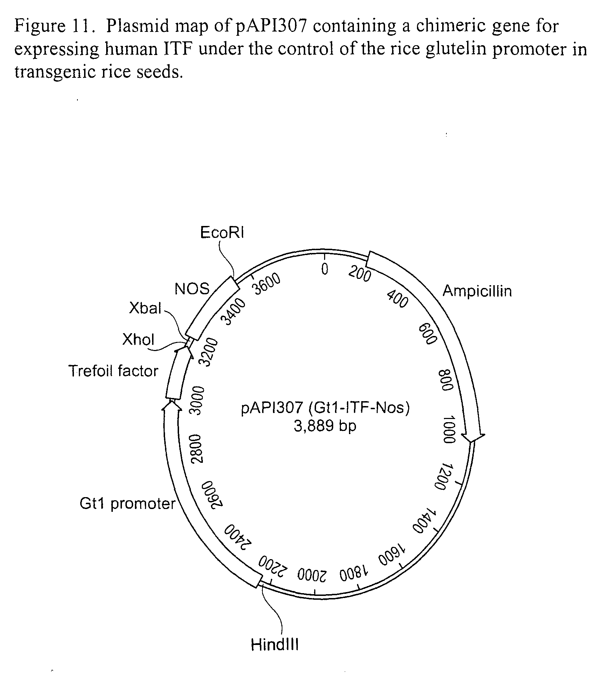

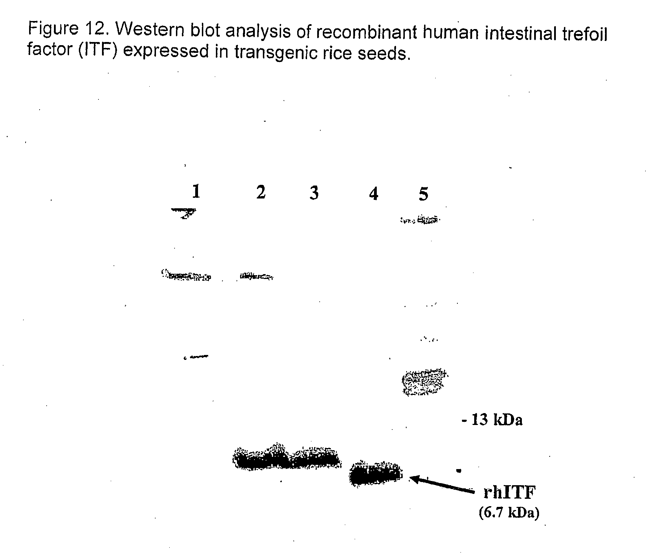

The ITF gene was codon optimized as shown in Figure 9, and synthesized by Operon Technologies (CA, USA) (SEQ ID NO: 5). For expression of ITF in rice seeds, the codon optimized gene was operably linked to the rice endosperm specific glutelin (Gt1) promoter, Gt1 signal peptide and NOS terminator in pAPI307 (Figure 11), and to the rice endosperm specific globulin (Glb) promoter, Glb signal peptide and NOS terminator in pAPI269 (Figure 10). The transgenic plant expressing ITF was generated, and plant-generated recombinant ITF was detected as shown in Figure 12 and as exemplified herein.

### D. Western blot analysis for all growth factors

Both untransformed (rice variety Taipei 309) and transgenic rice seeds (∼10 pooled R1 seed from individual transgenic plants expressing either EGF, IGF-I or ITF) were ground in 1 ml of 0.35 M NaCl in phosphate buffered saline (PBS), pH 7.4, using an ice-cold mortar and pestle. The resulting extract was centrifuged at 14,000 rpm at 4°C for 10 min. Cleared supernatant was collected and -20 ug of this soluble protein extract was re-suspended in sample loading buffer, and loaded onto a pre-cast 10-20% polyacrylamide tricine gel (Novex) and subjected to SDS-PAGE. After electrophoresis, the gel was blotted to a 0.45 µm nitrocellulose membrane. The membrane was blocked with 5% non-fat dry milk in PBS pH 7.4 for 2 hrs followed by three washes with PBS for 10 min each. A primary rabbit polyclonal antibody prepared against EGF(Sigma), IGF-I (Sigma) or ITF (GI Company) was used at 1:2000 dilution in PBS. Bands were developed using goat anti-rabbit antibody coupled to the BCIP/NBT substrate system (Sigma). Results are shown in Figures 4, 8 and 12, respectively.

### Comparative Example 2: Expression of recombinant human lactoferrin

### A. An Expression Vector For Human Lactoferrin Expression In Transgenic Rice

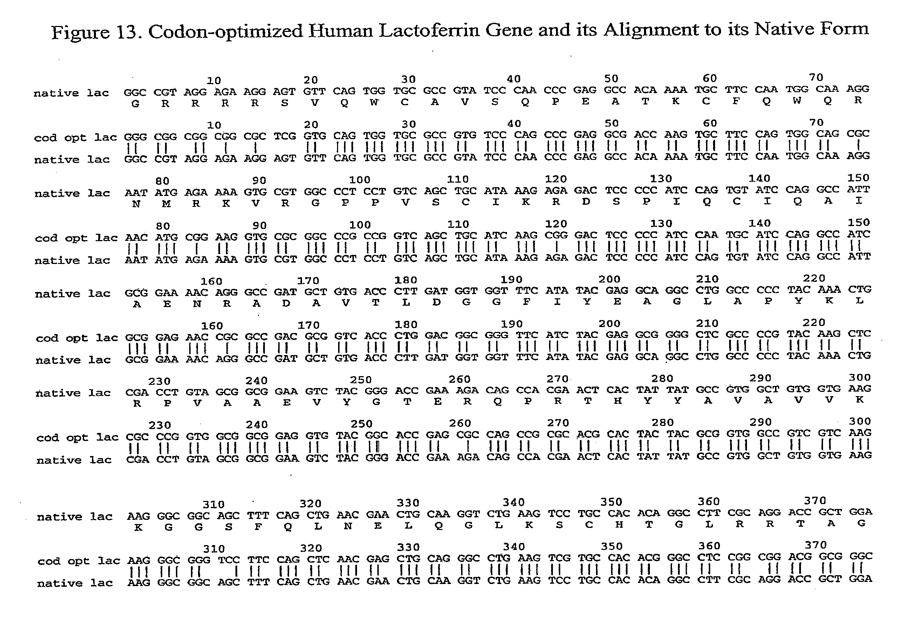

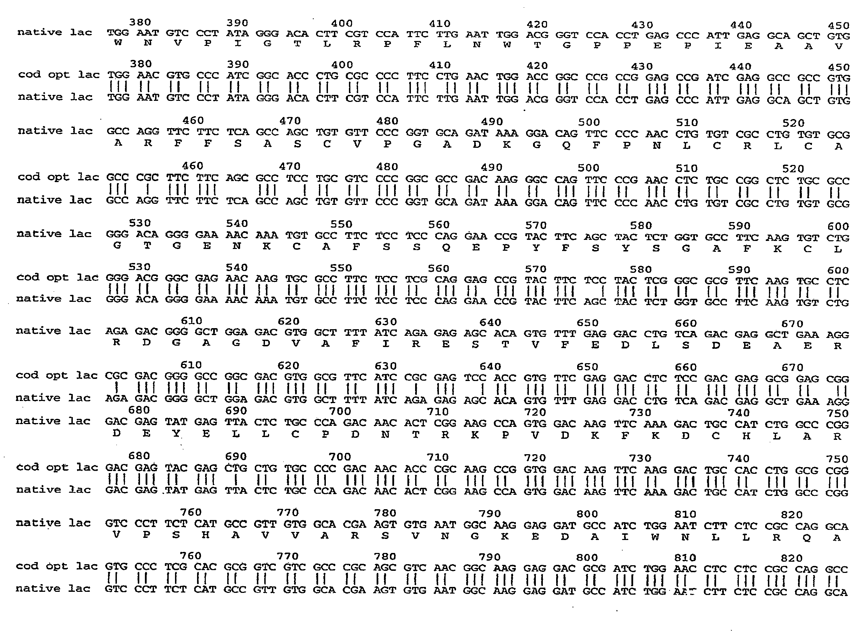

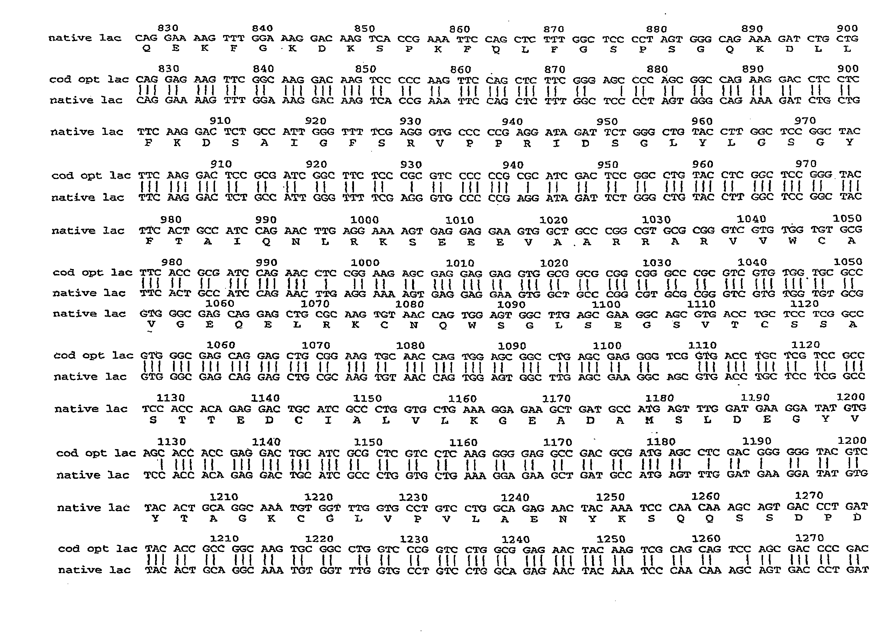

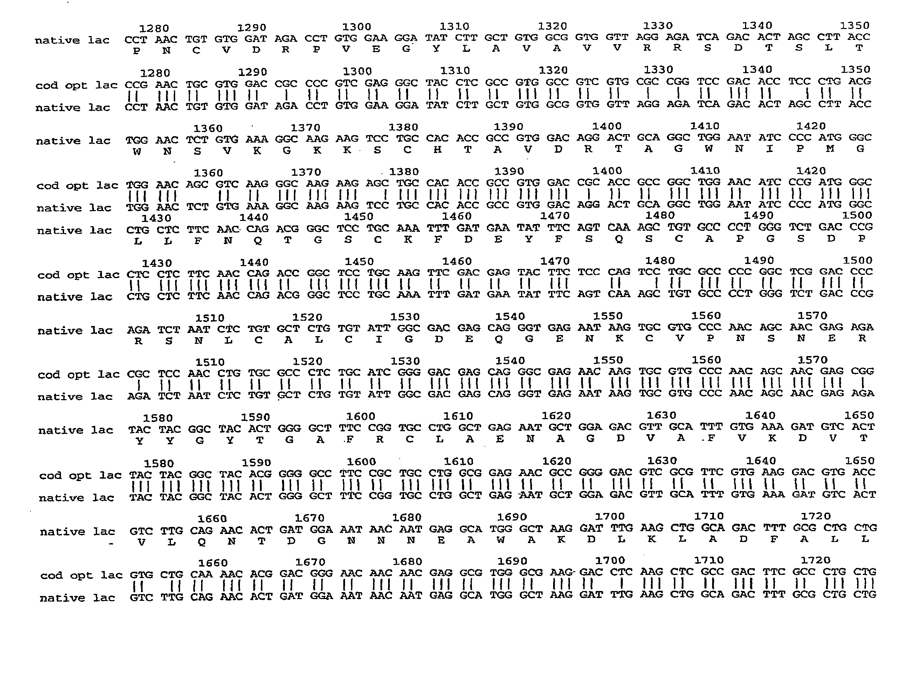

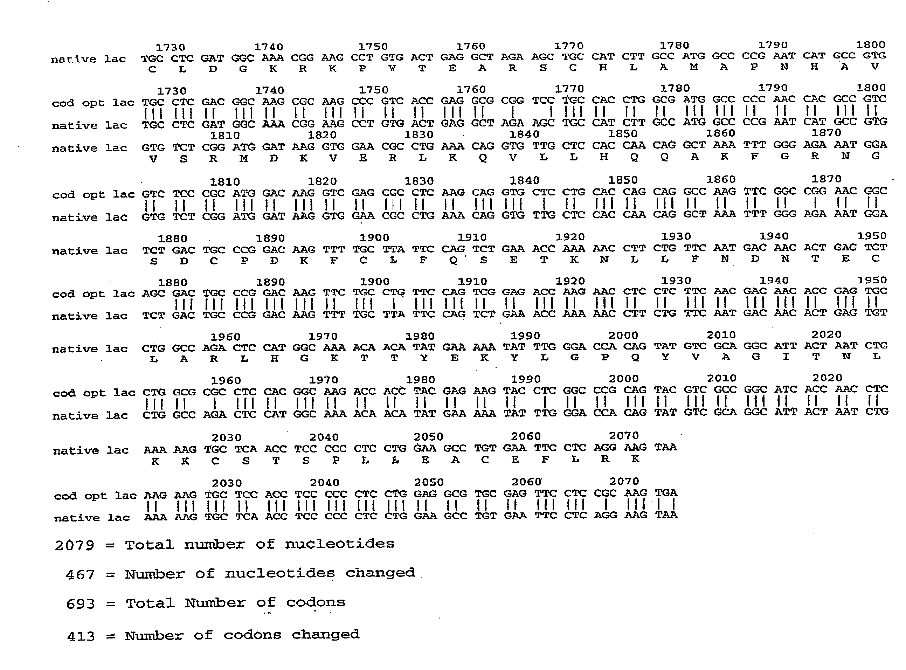

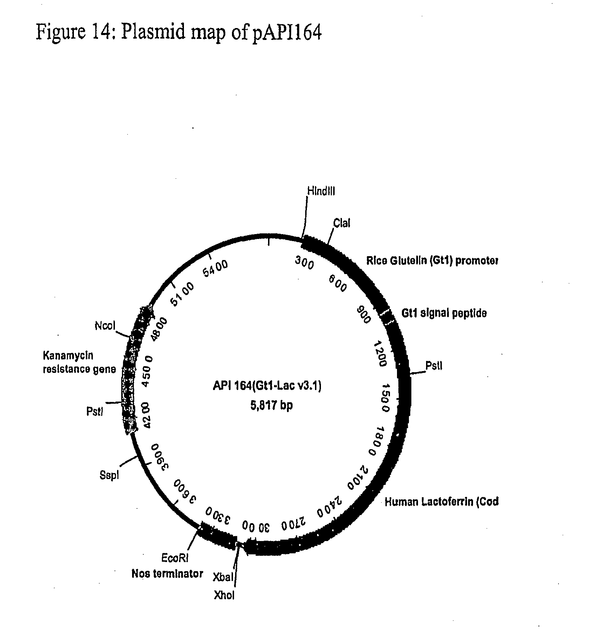

Complete nucleotide sequence of human mammary gland lactoferrin was codon optimized and synthesized by Operon Technologies (CA, USA). Human milk lactoferrin gene (Genbank accession number: HSU07642) was re-synthesized with codons most frequently used in translation of rice seed proteins in order to obtain optimal level of expression (Figure 13). Although numbers of codons changed accounted for 22.46% of the entire sequence, amino acid composition remains identical to native human lactoferrin. The plasmid containing the codon-optimized gene was called Lac-ger. Lac-ger was digested with Smal/Xhol and the fragment containing the lactoferrin gene was cloned into pAPI141 that was partially digested with Nael and completely digested with Xhol. For expression of hLF in rice seeds, the codon-optimized gene was operably linked to the rice endosperm-specific glutelin (Gt1) promoter and NOS terminator. The resulting plasmid was designated pAPI164 (Figure 14).

### B. Production system

Rice variety Taipei 309 (Oryza sativa, Japonica) was selected as the production system for recombinant human lactoferrin (rhLF) and transgenic rice events were generated by the particle bombardment of embryogenic rice calli with the plasmid pAPI164 and a companion marker plasmid containing the hygromycin phosphotransferase gene as a selectable marker. Fully developed, fertile rice plants were obtained by this procedure.

### C. High Level Protein Expression of recombinant human lactoferrin in rice grain

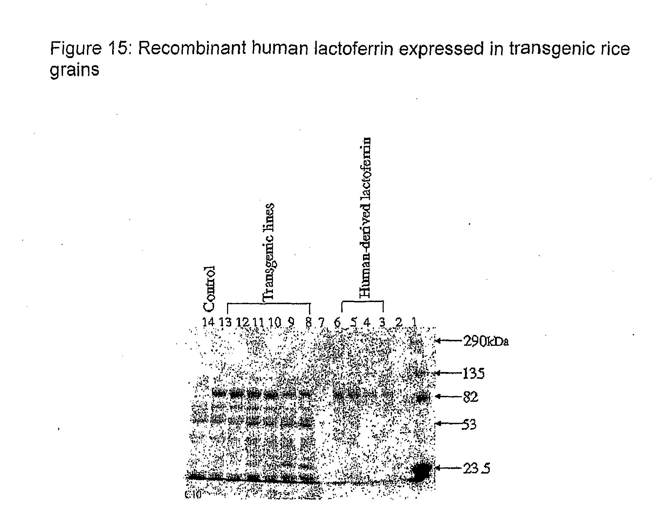

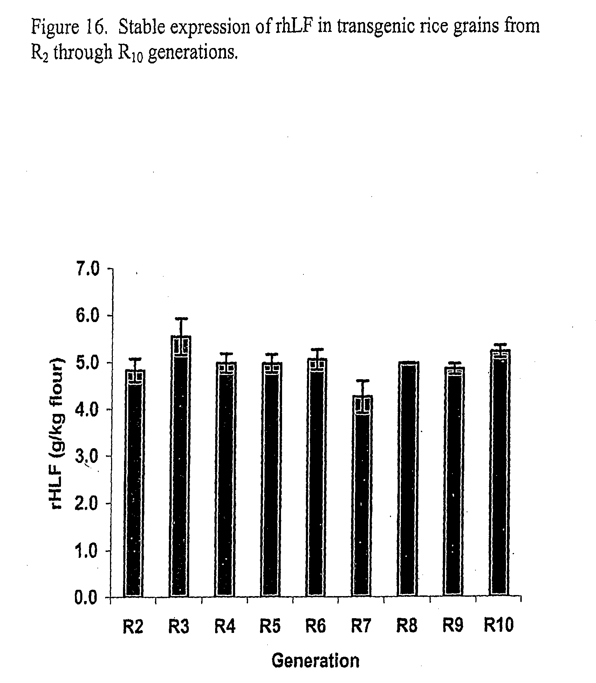

Expression of recombinant human lactoferrin was under the control of the seed maturation-specific promoter Gt1. The high level expression of recombinant human lactoferrin is evident in Figure 15 as independent transgenic rice events were screened. Total soluble proteins from mature rice seed extracts were run on Laemli gels and stained with Coomassie blue to visualize the proteins. An ∼80kD recombinant lactoferrin protein was obtained in all transgenic lines as indicated by the stained gel. Expression levels of recombinant human lactoferrin corresponded to 0.5% of seed weight. The stable expression of recombinant human lactoferrin was monitored for 10 generations. The expression level is maintained at approximately 0.5% of seed weight of brown rice (Figure 16).

### Example 3: Expression of recombinant human serum albumin in rice grain

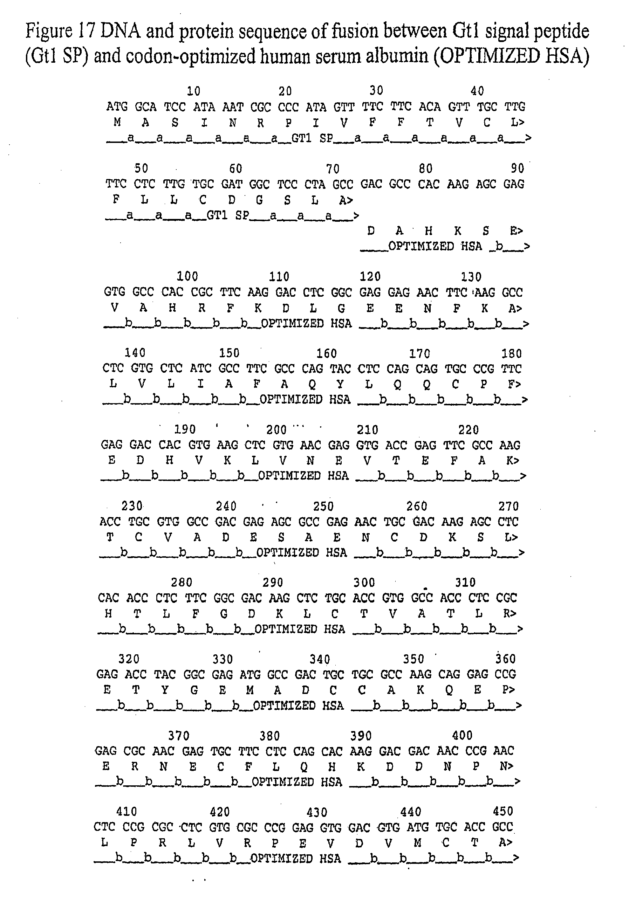

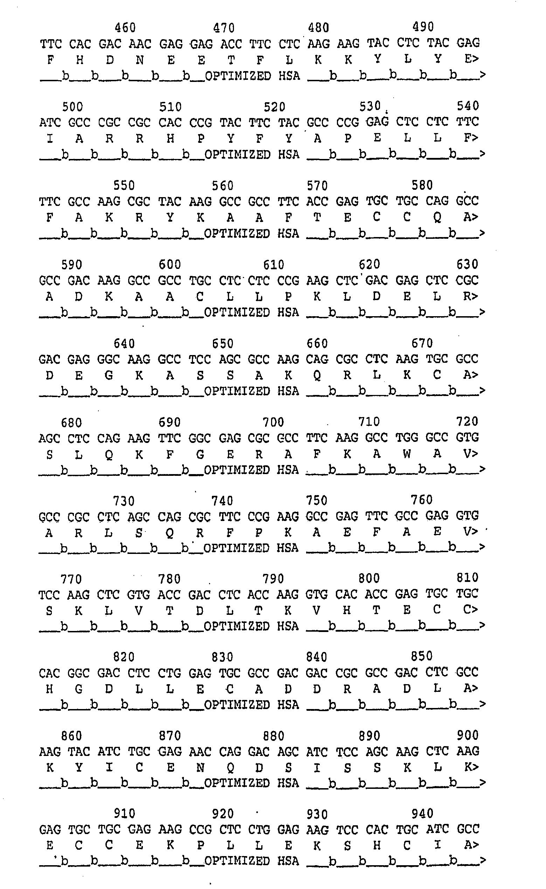

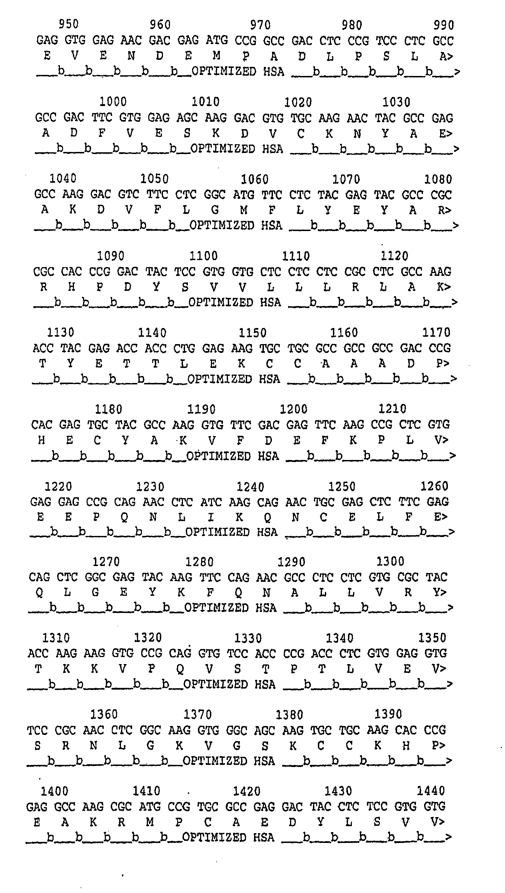

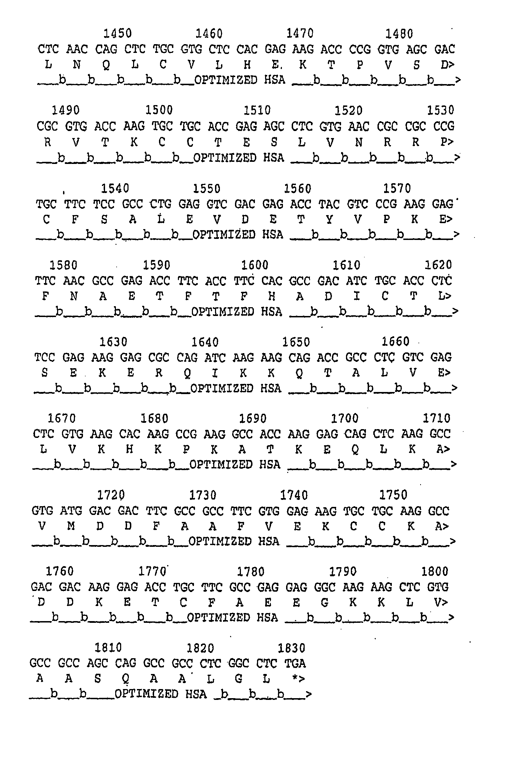

Protein sequences of human serum albumin (HSA) from various data bases were compared. The consensus sequence represented by accession number P02768 (Swiss-Prot) was used as a base for gene codon-optimization for suitable expression of human serum albumin in rice grain (Figure 17). Gene synthesis was carried out by Blue Heron (Seattle, WA) and the synthetic fragment was inserted into pUC based vector to create pUC-HSA. After confirmation of correct DNA and protein sequences, pUC-HSA was digested with Mlyl and Xhol. The fragment containing codon-optimized HSA gene was inserted into pAPI405, which had been precut with Nael and Xhol. Plasmid API405 was a derivative of pAPI141 which included Gt1 promoter, Gt1 signal sequence and a nos terminator.

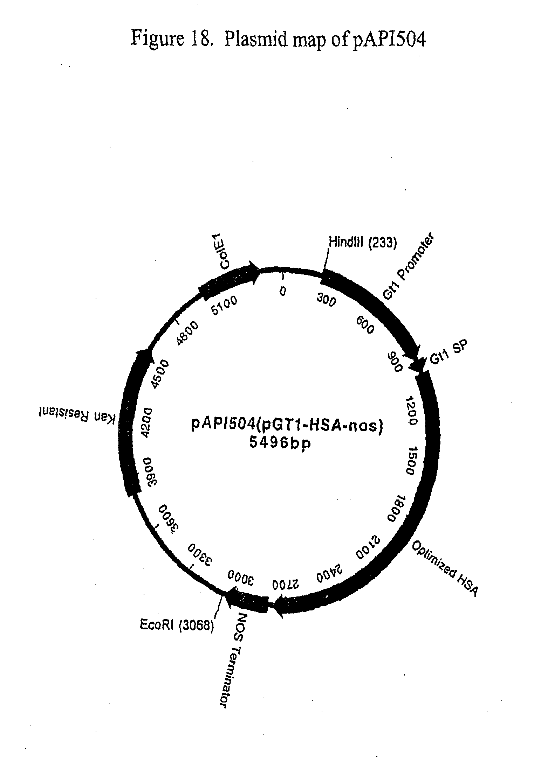

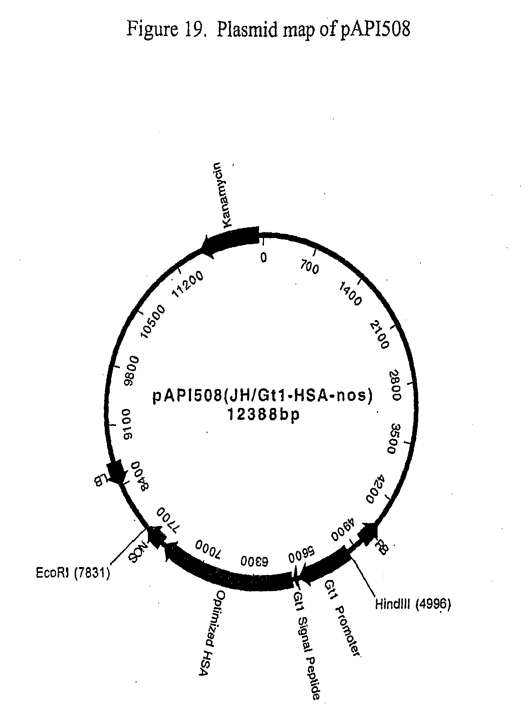

Insertion of Mlyl/Xhol fragment into pAPI405 resulted In pAPI504 (Figure 18). Plasmid API504 was then cleaved with HindIII and EcoRI. The HindIII/EcoRI fragment containing the entire expression cassette, Gt1 promoter, Gt1 signal sequence, codon-optimized HSA gene and nos terminator, was cloned into pJH2600 predigested with the same enzyme resulting in pAPI508 (Figure 19). Plasmid JH2600 was a shuttle vector between E coli and Agrobacterium. After DNA sequence verification, pAPI508 was moved into Agrobacterium AGL-1 for rice transformation. Plasmid API504 was also used via bombardment-based transformation following the procedure described previously. Upon transformation, transgenic plants were generated and were sent to greenhouse where transgenic R0 plants grew to maturity and set R1 seeds.

To monitor the expression of HSA in rice seeds, 10 R1 seeds from each R0 plant were extracted using 10 mL of extraction buffer (50 mM Tris-HCl pH 8.0. 150 mM NaCl). The supernatant was collected and the expression level from rice extracts was monitored by an ELISA (Bethyl Laboratories, Montgomery, TX). The results showed that the HSA expression level in rice transgenic seeds ranges from 0.01 to 0.85% of brown flour weight (0.1 to 8.5 grams/kg flour). The results of the eight events with highest expression levels are shown in the following table (Table 1).

**Table 1. Recombinant HSA expression level in top eight events**

| Line Number | Express Level (g/kg flour) |
|---|---|
| 508-3 | 5.5 |
| 508-17 | 8.0 |
| 508-71 | 8.7 |
| 508-73 | 4.0 |
| 508-77 | 8.5 |
| 508-83 | 8.5 |
| 508-101 | 3.0 |
| 508-113 | 4.0 |

### Comparative Example 4: Purification of recombinant human lactoferrin as cell culture media component

To prepare cell culture media supplemented with recombinant human lactoferrin, recombinant human lactoferrin was purified from rice flour. Transgenic rice line (164-12) expressing high levels of rhLF was selected. This line, now named as LF164, was planted two generations per year, alternating field planting in summer and greenhouse planting in winter. For protein purification, paddy rice expressing rhLF was de-hulled (Rice Mill, PS-160, Rimac, FL), and then ground to flour (average particle size of 100 mesh) using a hammer mill (8WA, Schutte-Buffalo, NY).

Protein extraction from transgenic flour was performed by mixing two kg of rice flour and 20 L of extraction buffer (0.02 M sodium phosphate pH 6.5 and 0.3 M sodium chloride) in a 50 L tank for 1 h. At the end of the mixing period, the suspension was allowed to settle overnight or centrifuged at 3750 rpm. In both cases, the supernatant was filtered through a plate and frame filter press (Ertel Alsop, 8S, NY) using M-05 and M-70 cellulose/perlite-based filters (Ertel Alsop, NY), respectively.

The filtrate containing rhLF and other rice flour soluble proteins was loaded onto an ion exchange column for further purification. An INDEX 200/500 process column (Amersham Pharmacia Biotech, NJ) packed with SP-Sepharose fast flow (Amersham Pharmacia Biotech, NJ) was used. The column was run at a linear flow rate of 150-200 cm/h. Packing, cleaning and testing of the resin bed was executed per manufacturer's instruction (HETP Test). The filtrate was loaded on to the resin at a linear velocity of 175 cm/h and washed with 0.02 M sodium phosphate buffer (pH 6.5) containing 0.3 M NaCl until the A₂₈₀ returned to baseline. Recombinant hLF was eluted using 20 mM sodium phosphate buffer (pH 6.5) containing 0.8 M NaCl. The washing and elution were performed at 200 cm/h and 150 cm/h, respectively.

A Centramate module (Pall Biopharmaceutical, MA) with 1 ft² 50 kDa polyethersulfone (Pall Biopharmaceutical, MA) membrane was used for concentration and desalting (ultrafiltration) of eluted hLF. The filtration was performed at a cross flow rate of approximately 1.5 L/min and an average transmembrane pressure of 10 psig. The eluted rhLF was concentrated and desalted to a final volume of 0.25 L and then lyophilized dry. Usually, about 3 grams of purified recombinant human lactoferrin was recovered from one kilogram of transgenic rice flour.

The recombinant human lactoferrin purified from rice flour is approximately 50% saturated with iron (partial-lactoferrin). The 50% saturated recombinant human lactoferrin was then made >90% iron saturated by iron up taking treatment, resulting in holo-lactoferrin and was made <10% iron saturated by acid treatment to remove bound iron resulting in apo-lactoferrin. The purified lactoferrins (holo-, partial- and apo-) were used as cell culture media components.

### Comparative Example 5: Cell culture media supplemented with recombinant human lactoferrin promotes cell growth

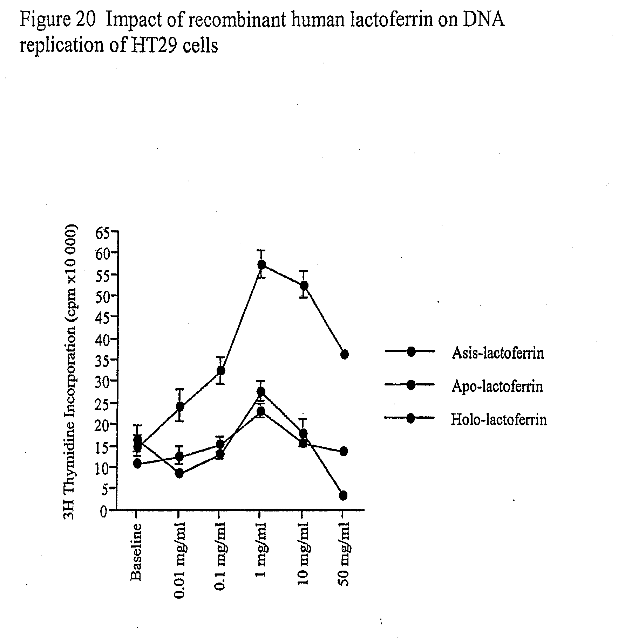

Recombinant human lactoferrin was added to minimal essential media (MEM) at various concentrations. This allows the test of growth factor effect of lactoferrin. The human colonic cancer cell line, HT-29, were maintained in MEM basic media containing 10% fetal calf serum (FCS). Lactoferrin samples were subsequently tested under serum-free conditions (MEM without supplementation of fetal calf serum). To assess the percentage of cells entering DNA synthesis, [³H]-thymidine (2 µCi/well) was included twenty four hours after the addition of lactoferrin samples and cells were left for a further 24 h. For each condition, the stimulatory or inhibitory effect of the lactoferrin solutions was measured in duplicate in six separate wells. Cell viability, determined by the ability to exclude 0.2% trypan blue, was greater than 90%. Proliferation assays reveal a typical 'bell shaped' dose response curve (Figure 20). Results demonstrated these responses to lactoferrin addition and show that maximal activity of lactoferrin was at a final concentration of 1 mg/ml, for all three forms of lactoferrin. Furthermore, of the three forms, the holo-lactoferrin showed the greatest proliferative activity (Figure 20).

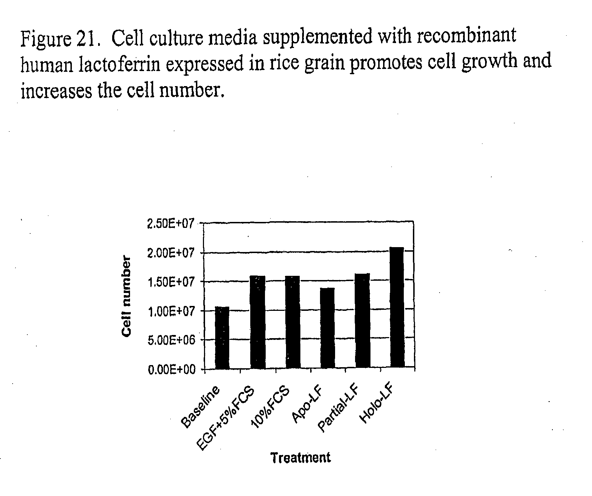

To determine cell viability, HT-29 Cells were seeded in 24-well plates at 5 x 10⁵ cells per well in MEM supplemented with 5% fetal calf serum (FCS); this resulted in an attached cell number of >95% per well 24h later (as assessed by trypsinization and direct counting using a Neubauer haemocytometer). At this time, the medium was changed in identically seeded wells to one containing MEM with 5 % FCS alone or MEM with 5% FCS plus EGF, or additional 5% FCS or various forms of Lactoferrin (i.e., apo-, partial- or holo-) at a concentration of 1 mg/ml for a further 48h. At the end of this period, cell numbers were assessed. To ensure that any cells present in the supernatant at this time were included in the total cell count, supernatants were collected, centrifuged at 13,000 rpm (12,000 g) for 5 min and added to the cells following trypsinization. Result shows that recombinant human lactoferrin derived from rice promote cell growth. There were twice as many cells in wells supplemented with holo-lactoferrin as that in wells without lactoferrin (Figure 21). Both thymidine incorporation and cell count experiments indicate that recombinant human lactoferrin from rice can be added as a component to cell culture media for fast cell growth (Figures 20 and 21).

### Comparative Example 6: Cell culture media supplemented with recombinant human lactoferrin promotes hybridoma cell growth

Recombinant human holo-lactoferrin was added to serum free media at various concentrations ranging from 5 to 250 mg/ml for hybridoma cell culture. Since the recombinant human lactoferrin is iron-saturated, holo-lactoferrin can provide iron for cell growth. The hybridoma cell line AE1 (ATCC) was maintained in DMEM basic media containing 5% fetal bovine serum (FBS). Lactoferrin was tested under serum-free conditions (AFM6, KC Bio, Kansas) without supplementation of fetal bovine serum. The cells were subcultured from 5% FBS to serum free media over multiple passages. At each subculture, the cells were analyzed for total cell count and viability. In order to demonstrate that recombinant human lactoferrin can replace transferrin derived from animal source, transferrin of equal concentration was added to AFM6 media as the control. The results demonstrated that the recombinant human holo-lactoferrin can be used to replace animal-derived transferrin in cell culture media for hybridoma cell growth.

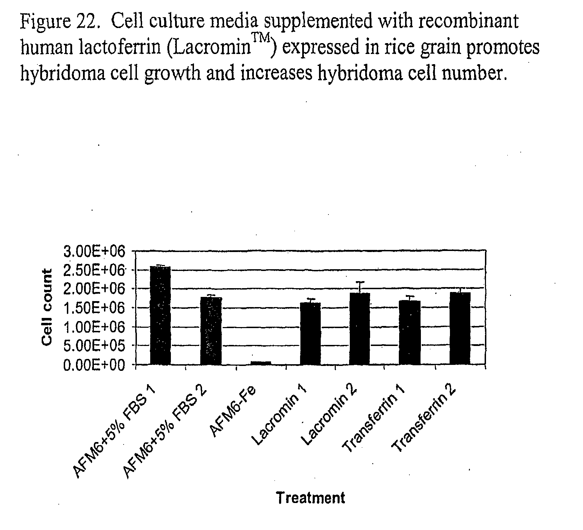

To determine the ability of holo-lactoferrin to promote cell growth, AE1 cells were sub-cultured from media containing 5% FBS to serum free media in T25 stationary flasks. The serum free media was supplemented with either animal derived transferrin; which is commonly used in the industry, or recombinant human holo-lactoferrin (LACROMIN™). This allows for a direct comparison between recombinant human holo-lactoferrin and animal derived transferrin. Each protein test was performed in triplicate with seeding densities of 5 X10⁵ cells per ml. To the subculture, the cells were collected by centrifugation and then seeded into fresh media with identical concentrations of either transferrin or recombinant human holo-lactoferrin (LACROMIN™). Each flask was analyzed on day 1 and day 3, following the subculture, for cell number and viability. An average cell count is given in Figure 22 for two passages. As can be seen, the cell viability and cell number when using recombinant human holo-lactoferrin are much higher than that without lactoferrin and equivalent to that of animal derived transferrin, indicating that plant-derived recombinant human holo-lactoferrin can promote hybridoma cell growth and replace animal derived transferrin.

The same analysis was carried out with another hybridoma cell line, L243. As can be seen in Table 2. LACROMIN^{™} (rhLF) can promote cell growth as as well as human transferrin (TF). L243 is also an IgG producing cell line. Samples were taken for IgG production. In medium containing LACROMIN^{™}, more IgG is produced than in medium containing transferrin.

**Table 2. Effect of human transferrin (TF) and recombinant human lactoferrin (rhLF) on hybridoma growth and monoclonal antibody production.**

| | TF/rhLF | Cell count (X10⁶) | | IgG level (ug/ml) | |
|---|---|---|---|---|---|
| Treatment | (ug/ml) | Mean | SD | Mean | SD |
| Control | 0 | 0.39 | 0.18 | 15.78 | 5.63 |
| TF | 5 | 0.70 | 0.25 | 34.10 | 8.44 |
| rhLF | 5 | 0.63 | 0.10 | 43.58 | 8.24 |
| TF | 25 | 0.78 | 0.24 | 31.93 | 7.10 |
| rhLF | 25 | 0.81 | 0.20 | 36.10 | 7.54 |
| TF | 100 | 0.86 | 0.33 | 28.58 | 5.82 |
| rhLF | 100 | 0.78 | 0.26 | 29.73 | 4.28 |
| 5% FBS | 0 | 0.98 | 0.12 | 23.88 | 5.50 |

### Example 7: Purification and characterization of recombinant human serum albumin

To purify rHSA from rice grain, rice grain expressing rHSA was dehusked, ground to flour and mixed for 30 min. with rHSA extraction buffer (Sodium Acetate, pH 4.9). After clarification by filtration, the filtrate was loaded onto an anion-exchange column (Q-Sepharose; GE Healthcare) at a rate of approximately 150 cm/hr. Once loaded, the resin was then washed with equilibration buffer to remove any unbound protein. The eluted rHSA is approximately 90% pure as analyzed by SDS-PAGE. Blue-Sepharose (GE Healthcare) can then be used to increase the purity from 90% to approximately 98% based on scanning analysis of SDS-PAGE gels. The rHSA is then concentrated and desalted via ultrafiltration prior to freeze drying.

Purified rHSA was subjected to a range of biochemical and biophysical characterizations for comparison to pHSA (Plasma-derived HSA) (Table 3). These tests indicate that rHSA expressed in rice is equivalent to pHSA.

**Table 3. Properties of rHSA as compared to pHSA**

| **Property** | **pHSA** | **rHSA** |
|---|---|---|
| N-terminal sequence | DAHKSE | DAHKSE |
| Glycosylation | None | None |
| SDS-PAGE/Western | ∼66 kDa | ∼66 kDa |
| Molecular mass (MALDI) | 66.9-68.1 kDa | 66.8-67.9 kDa |
| Isoelectric focusing point | pl 5.3 | pl 5.3 |
| Ligand binding | Yes | Yes |
| Thermal stability | Midpoint 65°C | Midpoint 65°C |
| Esterase activity | Yes | Yes |
| Protease sensitivity | Same as each other | Same as each other |

### Example 8: Cell culture media supplemented with recombinant human serum albumin promotes hybridoma cell growth in reduced -serum media.

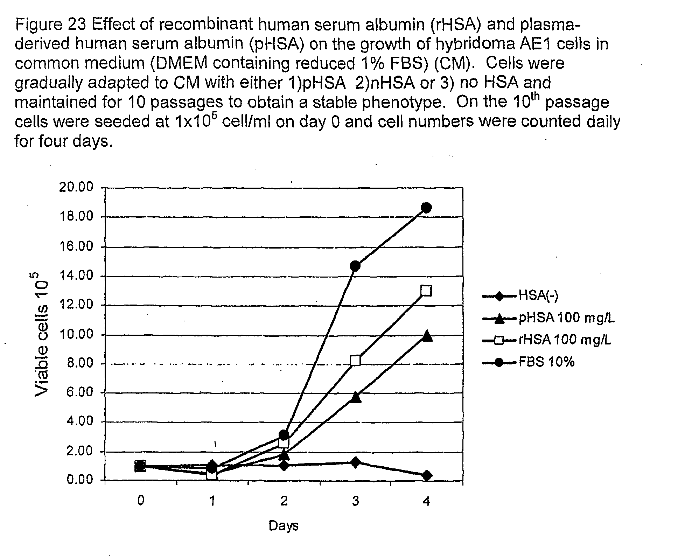

The effectiveness of recombinant human serum albumin from rice (rHSA) to stimulate cell growth similar to plasma derived human serum albumin (pHSA) was tested. Basal medium DMEM supplemented with 10 µg/ml insulin, 5.5 µg/ml transferrin, 6.7 ng/ml sodium selenite, 0.20 mg/ml ethanolamine and 2 mM Glutamax (Gibco) was used as common medium (CM). Hybridoma AE1 stocks in CM + 10% fetal bovine serum (FBS), were divided into four lineages: 1) CM +10% FBS as positive control 2) CM without HSA as negative control 3) CM + 100 mg/L rHSA as experimental, and 4) CM +100mg/L plasma-derived HSA (pHSA) as comparative control. Each lineage was then gradually adapted to 1 % FBS (reduced serum) by sequential passage of 1x10⁵ cell/ml into medium containing 50% reduced FBS. At 1% FBS, each lineage was subsequently passaged 10 times to allow for full adaptation to growth under reduced-serum (1% FBS) conditions. Figure 23 shows cell growth curves of the 4 lineages during the 10th passage under CM +1%FBS. It was found that AE1 hybridoma cells grew much faster and reached a higher density in CM rHSA than in CM without any HSA (Figure 23). It was also found that cells grown in media with rHSA supplementation grew equivalently or better than cells grown in media with plasma derived HSA. This result indicates that rHSA from rice is functionally equivalent to pHSA in enhancing hybridoma cell growth in reduced-serum media.

### Example 9: Cell culture media supplemented with recombinant human serum albumin promotes hybridoma cell growth in serum-free media.

In order to demonstrate that plant derived recombinant human serum albumin functions equivalently to albumin derived from animals to support cell growth, the growth of AE1 hybridoma cells in supplemented serum-free media (SFM) was tested. SFM was prepared that was supplemented with either recombinant human serum albumin or plasma derived human serum albumin at similar concentrations (100 mg/L). SFM without supplemental albumin was prepared as a negative control. This approach allows for direct comparison between plant derived recombinant human serum albumin (CELLASTIM™) and plasma derived human serum albumin in promoting cell growth. SFM consisted of DMEM/F12 basal media supplemented with 10 µg/ml insulin, 5.5 µg/ml transferrin, 6.7 ng/ml sodium selenite, 0.20 mg/ml ethanolamine and 2 mM Glutamax (Gibco). To reach serum-free growth conditions, AE1 Hybridoma cells (ATCC # HB-72) were first adapted to SFM without FBS by a serial adaptation procedure. Briefly, AE1 grown in SFM/10% FBS supplemented with recombinant human serum albumin or plasma derived human serum albumin or no HSA, were successively subcultured at 1x 10⁵ cells/ml into SFM with 50% reduced serum from the previous culture. The SFM/0.25% FBS cultures were subcultured directly into SFM without FBS. Six successive subcultures followed in SFM to allow cells to fully adapt to the respective SFM without FBS.

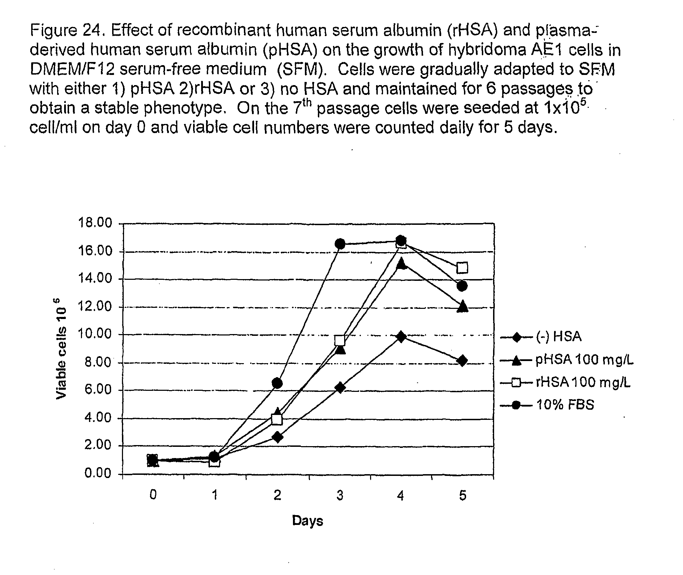

AE1 hybridoma cells grown for 6 passages in SFM containing 100 mg/L recombinant human serum albumin or 100 mg/L plasma derived human serum albumin or no HSA were sub-cultured 6-well plates at 1x10⁵ cells/ml. The number of viable cells and percent viability was determined daily to examine growth kinetics of the cultures. The subcultures were performed in duplicate to ensure an accurate viable cell count and analysis. Figure 24 shows that AE1 hybridoma cell grew faster and that the maximum number of viable cells was higher in medium supplemented with plant derived recombinant human serum albumin and equivalent to that of plasma derived human serum albumin. Thus, this example shows that plant derived recombinant human serum albumin can promote cell growth in serum-free media and replace animal derived albumin for use in tissue culture.

## Claims

1. A cell culture media supplement, comprising at least one plant-produced heterologous protein as a cell culture media component, wherein the protein is recombinant human serum albumin produced in a rice plant, that improves growth and productivity of cells compared to the use of media containing animal-derived human serum albumin when cultivating AE1 hybridoma cells, wherein the cell culture media supplement still contains other components from the purifying or isolating step(s) of the human serum albumin.

2. The cell culture media supplement of Claim 1, wherein the recombinant human serum albumin is produced in the rice plant seed and comprises about 3% or greater of the total soluble protein in the rice plant seed.

3. The cell culture media supplement of Claim 2, wherein the human serum albumin is expressed in the rice plant using an expression vector comprising a promoter derived from a gene encoding a maturation specific monocot plant storage protein, a first DNA sequence, operably linked to the promoter, encoding a monocot plant seed-specific signal sequence capable of targeting a polypeptide linked thereto to an endosperm cell, and a second DNA sequence, linked in translation frame with the first DNA sequence, encoding human serum albumin.

4. The cell culture media supplement of Claim 3, wherein the maturation specific monocot promoter is selected from rice glutelins, oryzins and prolamines, barley hordeins, wheat gliadins and glutelins, maize zeins and glutelins, oat glutelins, sorghum kafirins, millet pennisetins, and rye secalins.

5. The cell culture media supplement of Claim 1, wherein the recombinant human serum albumin is purified from the rice plant via Q-Sepharose anion exchange chromatography.

6. The cell culture media supplement of any of Claims 1 to 5, for use in cell culture, where the cell culture media is a reduced serum or serum free media, or a chemically defined media.

7. The cell culture media supplement of any of Claims 1 to 5, for use in cell culture, where the cell culture media is a complete media, basal media, or basal media supplemented with a cell culture media component.

8. The cell culture media supplement of any of Claims 1 to 5, for use in cell culture, where the cell culture media is derived exclusively from non-animal sources.

9. A cell culture medium, comprising the cell culture media supplement of claim 1.

10. Use of recombinant human serum albumin produced in a rice plant as a cell culture media supplement, wherein the recombinant human serum albumin improves growth and productivity of cells compared to the use of media containing animal-derived human serum albumin.

11. A method of cell culture, comprising the use of a cell culture media supplement comprising at least one plant-produced heterologous protein as a cell culture media component, wherein the protein is recombinant human serum albumin produced in a rice plant that improves growth and productivity of cells compared to the use of media containing animal-derived human serum albumin.

## Patentansprüche

1. Zellkulturmediumsupplement, umfassend mindestens ein Pflanzen-produziertes heterologes Protein als Zellkulturmediumsbestandteil, wobei das Protein ein rekombinantes humanes Serumalbumin ist, welches in einer Reispflanze produziert wurde, welches das Wachstum und die Produktivität von Zellen im Vergleich zur Verwendung von Medium, welche humanes Serumalbumin aus Tieren enthält, verbessert, wenn AE1 Hybridomazellen kultiviert werden, wobei das Zellkulturmediumsupplement noch weitere Bestandteile aus den Aufreinigungs-oder Isolationsschritt(en) des humanen Serumalbumins enthält.

2. Zellkulturmediumsupplement nach Anspruch 1, wobei das rekombinante humane Serumalbumin im Samen der Reispflanze produziert wird und etwa 3% oder mehr des gesamten löslichen Proteins in dem Reispflanzensamen umfaßt.

3. Zellkulturmediumsupplement nach Anspruch 2, wobei das humane Serumalbumin in der Reispflanze exprimiert wird unter Verwendung eines Expressionsvektors, welcher einen Promotor abgeleitet von einem Gen, welches ein reifungsspezifisches Speicherprotein einkeimblättriger Pflanzen kodiert, eine erste DNA-Sequenz, welche funktionsfähig an einen Promotor angeschlossen ist, welche eine Signalsequenz, die für den Samen einer einkeimblättrigen Pflanze spezifisch ist und welche befähigt ist, ein daran gekoppeltes Polypeptid an eine Endospermzelle zu zielen, kodiert und eine zweite DNA Sequenz, welche in den Translationsrahmen der ersten DNA Sequenz gekoppelt ist und humanes Serumalbumin kodiert, umfaßt.

4. Zellkulturmediumsupplement nach Anspruch 3, wobei der reifungsspezifische Promotor einkeimblättriger Pflanzen ausgewählt ist aus Reisglutelinen, Oryzinen und Prolaminen, Gerstenhordeine, Weizengliadine und -gluteline, Maiszeine und -gluteline, Hafergluteline, Sorghumkafirin, Hirsepennisetine und Roggensecaline.

5. Zellkulturmediumsupplement nach Anspruch 1, wobei das rekombinant humane Serumalbumin aus der Reispflanze mittels Q-Sepharose-Anionaustausch-Chromatographie gereinigt wird

6. Zellkulturmediumsupplement nach einem der Ansprüche 1 bis 5, zur Verwendung in Zellkultur, wobei das Zellkulturmedium ein Medium mit reduziertem Serum oder ein serumfreies Medium oder ein chemisch definiertes Medium ist.

7. Zellkulturmediumsupplement nach einem der Ansprüche 1 bis 5, zur Verwendung in Zellkultur, wobei das Zellkulturmedium ein Komplettmedium, ein Basalmedium, oder ein Basalmedium, welches mit Bestandteilen eines Zellkulturmediums supplementiert wurde, ist.

8. Zellkulturmediumsupplement nach einem der Ansprüche 1 bis 5, zur Verwendung in der Zellkultur, wobei das Zellkulturmedium ausschließlich aus nichttierischen Quellen stammt.

9. Zellkulturmedium, umfassend das Zellkulturmediumsupplement nach Anspruch 1.

10. Verwendung eines rekombinanten humanen Serumalbumins, welches in einer Reispflanze produziert wurde, als ein Zellkulturmediumsupplement, wobei das rekombinante humane Serumalbumin das Wachstum und die Produktivität der Zellen im Vergleich zur Verwendung von einem Medium, welches aus Tieren gewonnenes humanes Serumalbumin enthält, verbessert.

11. Verfahren zur Kultivierung von Zellen, umfassend das Verwenden eines Zellkulturmediumsupplements, umfassend mindestens ein in Pflanzen hergestelltes heterologes Protein als ein Bestandteil des Zellkulturmediums, wobei das Protein rekombinantes humanes Serumalbumin ist, welches in einer Reispflanze produziert wurde und welches Wachstum und Produktivität der Zellen im Vergleich zur Verwendung von einem Medium, welches aus Tieren stammendes humanes Serumalbumin enthält, verbessert.

## Revendications

1. Complément de milieu de culture cellulaire, comprenant au moins une protéine hétérologue produite par une plante en tant que composant de milieu de culture cellulaire, dans lequel la protéine est une sérumalbumine humaine recombinante produite dans un plant de riz, qui améliore la croissance et la productivité des cellules par rapport à l'utilisation de milieux contenant une sérumalbumine humaine dérivée d'un animal, quand des cellules d'hybridome AE1 sont cultivées, dans lequel le complément de milieu de culture cellulaire contient encore d'autres composants provenant de l'étape ou des étapes de purification ou d'isolement de la sérumalbumine humaine.

2. Complément de milieu de culture cellulaire selon la revendication 1, dans lequel la sérumalbumine humaine recombinante est produite dans la semence d'un plant de riz et comprend environ 3% ou plus de la protéine soluble totale dans la semence du plant de riz.

3. Complément de milieu de culture cellulaire selon la revendication 2, dans lequel la sérumalbumine humaine est exprimée dans le plant de riz au moyen d'un vecteur d'expression comprenant un promoteur dérivé d'un gène codant une protéine de réserve de plante monocotylédone spécifique de la maturation, une première séquence d'ADN, liée de manière fonctionnelle au promoteur, codant une séquence signal spécifique de la semence d'une plante monocotylédone capable de vectoriser un polypeptide lié à cette séquence vers une cellule d'endosperme, et une seconde séquence d'ADN, liée dans le cadre de traduction à la première séquence d'ADN, codant la sérumalbumine humaine.

4. Complément de milieu de culture cellulaire selon la revendication 3, dans lequel le promoteur de monocotylédone spécifique de la maturation est choisi parmi les glutélines, les oryzines et les prolamines de riz, les hordéines d'orge, les gliadines et les glutélines de blé, les zéines et les glutélines de maïs, les glutélines d'avoine, les cafirines de sorgho, les pennisétines de millet et les sécalines de seigle.

5. Complément de milieu de culture cellulaire selon la revendication 1, dans lequel la sérumalbumine humaine recombinante est purifiée à partir du plan de riz par chromatographie d'échange anionique sur Q-Sepharose.

6. Complément de milieu de culture cellulaire selon l'une quelconque des revendications 1 à 5, pour une utilisation dans une culture cellulaire, le milieu de culture cellulaire étant un milieu à teneur réduite en sérum ou exempt de sérum, ou un milieu chimiquement défini.

7. Complément de milieu de culture cellulaire selon l'une quelconque des revendications 1 à 5, pour une utilisation dans une culture cellulaire, le milieu de culture cellulaire étant un milieu complet, un milieu de base ou un milieu de base complété avec un composant de milieu de culture cellulaire.

8. Complément de milieu de culture cellulaire selon l'une quelconque des revendications 1 à 5, pour une utilisation dans une culture cellulaire, le milieu de culture cellulaire dérivant exclusivement de sources non animales.

9. Milieu de culture cellulaire, comprenant le complément de milieu de culture cellulaire selon la revendication 1.

10. Utilisation d'une sérumalbumine humaine recombinante produite dans un plant de riz comme complément de milieu de culture cellulaire, dans laquelle la sérumalbumine humaine recombinante améliore la croissance et la productivité des cellules par rapport à l'utilisation de milieux contenant une sérumalbumine humaine dérivée d'un animal.

11. Procédé de culture cellulaire comprenant l'utilisation d'un complément de milieu de culture cellulaire comprenant au moins une protéine hétérologue produite par une plante en tant que composant de milieu de culture cellulaire, dans lequel la protéine est une sérumalbumine humaine recombinante produite dans un plant de riz, qui améliore la croissance et la productivité des cellules par rapport à l'utilisation de milieux contenant une sérumalbumine humaine dérivée d'un animal.


### SEQUENCE LISTING

<110> VENTRIA BIOSCIENCE
<120> Components of Cell Culture Media Produced from Plant Cells
<130> A7662EPCA
<140> EP06774198.3
   <141> 2007-12-31
<150> US60/694,236
   <151> 2005-06-28
<150> PCT/US2006/025195
   <151> 2006-06-27
<160> 19
<170> PatentIn version 3.3
<210> 1
   <211> 162
   <212> DNA
   <213> Artificial
<220>
   <223> Synthetic codon optimized (egfactor) sequence
<220>
   <221> CDS
   <222> (1)..(159)
<400> 1
   aac tcc gac tcg gag tgc ccc ctc tcc cac gac ggt tac tgc ctc cac        48
   Asn Ser Asp Ser Glu Cys Pro Leu Ser His Asp Gly Tyr Cys Leu His
   1               5                   10                  15
   gac ggg gtc tgc atg tac atc gag gcc ctc gac aag tac gcc tgc aac        96
   Asp Gly Val Cys Met Tyr Ile Glu Ala Leu Asp Lys Tyr Ala Cys Asn
               20                  25                  30
   tgc gtc gtg ggc tac atc ggc gag cgg tgc cag tac cgc gac ctc aag       144
   Cys Val Val Gly Tyr Ile Gly Glu Arg Cys Gln Tyr Arg Asp Leu Lys
           35                  40                  45
   tgg tgg gag ctg cgc tga                                               162
   Trp Trp Glu Leu Arg
       50
<210> 2
   <211> 53
   <212> PRT
   <213> Artificial
<220>
   <223> synthetic Construct
<400> 2
   Asn Ser Asp Ser Glu Cys Pro Leu Ser His Asp Gly Tyr Cys Leu His
   1               5                   10                  15
   Asp Gly Val Cys Met Tyr Ile Glu Ala Leu Asp Lys Tyr Ala Cys Asn
               20                  25                  30
   Cys Val Val Gly Tyr Ile Gly Glu Arg Cys Gln Tyr Arg Asp Leu Lys
           35                  40                  45
   Trp Trp Glu Leu Arg
       50
<210> 3
   <211> 53
   <212> PRT
   <213> Artificial
<220>
   <223> Synthetic codon optimized (egfactor) sequence
<400> 3
   Asn Ser Asp Ser Glu Cys Pro Leu Ser His Asp Gly Tyr Cys Leu His
   1               5                   10                  15
   Asp Gly Val Cys Met Tyr Ile Glu Ala Leu Asp Lys Tyr Ala Cys Asn
               20                  25                  30
   Cys Val Val Gly Tyr Ile Gly Glu Arg Cys Gln Tyr Arg Asp Leu Lys
           35                  40                  45
   Trp Trp Glu Leu Arg
       50
<210> 4
   <211> 213
   <212> DNA
   <213> Artificial
<220>
   <223> Synthetic codon optimized (Insgfact) sequence
<220>
   <221> CDS
   <222> (1)..(210)
<400> 4
   ggc cca gag acc ctg tgc ggt gcg gag ctg gtg gac gcc ctc cag ttc       48
   Gly Pro Glu Thr Leu Cys Gly Ala Glu Leu Val Asp Ala Leu Gln Phe
   1               5                   10                  15
   gtc tgc ggg gac cgg ggc ttc tac ttc aac aag cca acg ggc tac ggg       96
   Val Cys Gly Asp Arg Gly Phe Tyr Phe Asn Lys Pro Thr Gly Tyr Gly
               20                  25                  30
   tcc tcc tcg cgc cgc gcc ccc cag acc ggc atc gtg gac gag tgc tgc      144
   Ser Ser Ser Arg Arg Ala Pro Gln Thr Gly Ile Val Asp Glu Cys Cys
           35                  40                  45
   ttc cgc tcc tgc gac ctc cgg cgg ctg gag atg tac tgc gcc cca ctc      192
   Phe Arg Ser Cys Asp Leu Arg Arg Leu Glu Met Tyr Cys Ala Pro Leu
       50                  55                  60
   aag ccc gcc aag agc gcc tga                                          213
   Lys Pro Ala Lys Ser Ala
                   65                  70
<210> 5
   <211> 70
   <212> PRT
   <213> Artificial
<220>
   <223> synthetic Construct
<400> 5
   Gly Pro Glu Thr Leu Cys Gly Ala Glu Leu Val Asp Ala Leu Gln Phe
   1               5                   10                  15
   Val Cys Gly Asp Arg Gly Phe Tyr Phe Asn Lys Pro Thr Gly Tyr Gly
               20                  25                  30
   Ser Ser Ser Arg Arg Ala Pro Gln Thr Gly Ile Val Asp Glu Cys Cys
           35                  40                  45
   Phe Arg Ser Cys Asp Leu Arg Arg Leu Glu Met Tyr Cys Ala Pro Leu
       50                  55                  60
   Lys Pro Ala Lys Ser Ala
   65                  70
<210> 6
   <211> 70
   <212> PRT
   <213> Artificial
<220>
   <223> Synthetic codon optimized (Insgfact) sequence
<400> 6
   Gly Pro Glu Thr Leu Cys Gly Ala Glu Leu Val Asp Ala Leu Gln Phe
   1               5                   10                  15
   Val Cys Gly Asp Arg Gly Phe Tyr Phe Asn Lys Pro Thr Gly Tyr Gly
               20                  25                  30
   Ser Ser Ser Arg Arg Ala Pro Gln Thr Gly Ile Val Asp Glu Cys Cys
           35                  40                  45
   Phe Arg Ser Cys Asp Leu Arg Arg Leu Glu Met Tyr Cys Ala Pro Leu
       50                  55                  60
   Lys Pro Ala Lys Ser Ala
   65                  70
<210> 7
   <211> 183
   <212> DNA
   <213> Artificial
<220>
   <223> Synthetic codon optimized (Trefoil) sequence
<220>
   <221> CDS
   <222> (1)..(180)
<400> 7
   gag gag tac gtc ggg ctc tcc gct aac caa tgc gcg gtc ccg gcc aag      48
   Glu Glu Tyr Val Gly Leu Ser Ala Asn Gln Cys Ala Val Pro Ala Lys
   1               5                   10                  15
   gac cgg gtg gac tgc ggc tac ccc cac gtg acg ccg aag gag tgc aac      96
   Asp Arg Val Asp Cys Gly Tyr Pro His Val Thr Pro Lys Glu Cys Asn
               20                  25                  30
   aac cgg ggc tgc tgc ttc gac tcc cgc atc cca ggc gtg ccg tgg tgc     144
   Asn Arg Gly Cys Cys Phe Asp Ser Arg Ile Pro Gly Val Pro Trp Cys
           35                  40                  45
   ttc aag ccc ctc acc cgc aag acg gag tgc acg ttc tga                 183
   Phe Lys Pro Leu Thr Arg Lys Thr Glu Cys Thr Phe
       50                  55                  60
<210> 8
   <211> 60
   <212> PRT
   <213> Artificial
<220>
   <223> synthetic Construct
<400> 8
   Glu Glu Tyr Val Gly Leu Ser Ala Asn Gln Cys Ala Val Pro Ala Lys
   1               5                   10                  15
   Asp Arg Val Asp Cys Gly Tyr Pro His Val Thr Pro Lys Glu Cys Asn
               20                  25                  30
   Asn Arg Gly Cys Cys Phe Asp Ser Arg Ile Pro Gly Val Pro Trp Cys
           35                  40                  45
   Phe Lys Pro Leu Thr Arg Lys Thr Glu Cys Thr Phe
       50                  55                  60
<210> 9
   <211> 60
   <212> PRT
   <213> Artificial
<220>
   <223> synthetic codon optimized (Trefoil) sequence
<400> 9
   Glu Glu Tyr Val Gly Leu Ser Ala Asn Gln Cys Ala Val Pro Ala Lys
   1               5                   10                  15
   Asp Arg Val Asp Cys Gly Tyr Pro His Val Thr Pro Lys Glu Cys Asn
               20                  25                  30
   Asn Arg Gly Cys Cys Phe Asp Ser Arg Ile Pro Gly Val Pro Trp Cys
   35 40 45
   Phe Lys Pro Leu Thr Arg Lys Thr Glu Cys Thr Phe
   50 55 60
<210> 10
   <211> 159
   <212> DNA
   <213> Homo sapiens
<400> 10
   aatggtgact ctgaatgtcc cctgtcccac gatgggtact gcctccatga tggtgtgtgc 60
   atgtatattg aagcattgga caagtatgca tgcaactgtg ttgttggcta catcggggag 120
   cgatctcagt accgagacct gaagtggtgg gaactgcgc 159
<210> 11
   <211> 209
   <212> DNA
   <213> Homo sapiens
<400> 11
   ggaccggaga cgctctgcgg ggctgagctg gtggatgctc ttcagttcgt gtgtggagac 60
   aggggctttt atttcaacaa gcccacaggg tatggctcca gcagtcggag ggcgcctcag 120
   acaggcatcg tggatgagtg ctgcttccgg agctgtgatc taaggaggct ggagatgtat 180
   tgcgcacccc tcaagcctgc caagtcagc 209
<210> 12
   <211> 183
   <212> DNA
   <213> Homo sapiens
<400> 12
   gaggagtacg tgggcctgtc tgcaaaccag tgtgccgtgc cggccaagga cagggtggac 60
   tgcggctacc cccatgtcac ccccaaggag tgcaacaacc ggggctgctg ctttgactcc 120
   aggatccctg gagtgccttg gtgtttcaag cccctgacta ggaagacaga atgcaccttc 180
   tga 183
<210> 13
   <211> 2079
   <212> DNA
   <213> Homo sapiens
<220>
   <221> CDS
   <222> (1)..(2076)
<400> 13
   ggc cgt agg aga agg agt gtt cag tgg tgc gcc gta tcc caa ccc gag        48
   Gly Arg Arg Arg Arg Ser Val Gln Trp Cys Ala Val Ser Gln Pro Glu
   1               5                   10                  15
   gcc aca aaa tgc ttc caa tgg caa agg aat atg aga aaa gtg cgt ggc      96
   Ala Thr Lys Cys Phe Gln Trp Gln Arg Asn Met Arg Lys Val Arg Gly
               20                  25                  30
   cct cct gtc agc tgc ata aag aga gac tcc ccc atc cag tgt atc cag     144
   Pro Pro Val Ser Cys Ile Lys Arg Asp Ser Pro Ile Gln Cys Ile Gln
           35                  40                  45
   gcc att gcg gaa aac agg gcc gat gct gtg acc ctt gat ggt ggt ttc     192
   Ala Ile Ala Glu Asn Arg Ala Asp Ala Val Thr Leu Asp Gly Gly Phe
       50                  55                  60
   ata tac gag gca ggc ctg gcc ccc tac aaa ctg cga cct gta gcg gcg     240
   Ile Tyr Glu Ala Gly Leu Ala Pro Tyr Lys Leu Arg Pro Val Ala Ala
   65                  70                  75                  80
   gaa gtc tac ggg acc gaa aga cag cca cga act cac tat tat gcc gtg     288
   Glu Val Tyr Gly Thr Glu Arg Gln Pro Arg Thr His Tyr Tyr Ala Val
                   85                  90                  95
   gct gtg gtg aac aag ggc ggc agc ttt cag ctg aac gaa ctg caa ggt     336
   Ala Val Val Asn Lys Gly Gly Ser Phe Gln Leu Asn Glu Leu Gln Gly
               100                 105                 110
   ctg aag tcc tgc cac aca ggc ctt cgc agg acc gct gga tgg aat gtc     384
   Leu Lys Ser Cys His Thr Gly Leu Arg Arg Thr Ala Gly Trp Asn Val
           115                 120                 125
   cct ata ggg aca ctt cgt cca ttc ttg aat tgg acg ggt cca cct gag     432
   Pro Ile Gly Thr Leu Arg Pro Phe Leu Asn Trp Thr Gly Pro Pro Glu
       130                 135                 140
   ccc att gag gca gct gtg gcc agg ttc ttc tca gcc agc tgt gtt ccc     480
   Pro Ile Glu Ala Ala Val Ala Arg Phe Phe Ser Ala Ser Cys Val Pro
   145                 150                 155                 160
   ggt gca gat aaa gga cag ttc ccc aac ctg tgt cgc ctg tgt gcg ggg     528
   Gly Ala Asp Lys Gly Gln Phe Pro Asn Leu Cys Arg Leu Cys Ala Gly
                   165                 170                 175
   aca ggg gaa aac aaa tgt gcc ttc tcc tcc cag gaa ccg tac ttc agc     576
   Thr Gly Glu Asn Lys Cys Ala Phe Ser Ser Gln Glu Pro Tyr Phe Ser
               180                 185                 190
   tac tct ggt gcc ttc aag tgt ctg aga gac ggg gct gga gac gtg gct     624
   Tyr Ser Gly Ala Phe Lys Cys Leu Arg Asp Gly Ala Gly Asp Val Ala
           195                 200                 205
   ttt atc aga gag agc aca gtg ttt gag gac ctg tca gac gag gct gaa     672
   Phe Ile Arg Glu Ser Thr Val Phe Glu Asp Leu Ser Asp Glu Ala Glu
       210                 215                 220
   agg gac gag tat gag tta ctc tgc cca gac aac act cgg aag cca gtg     720
   Arg Asp Glu Tyr Glu Leu Leu Cys Pro Asp Asn Thr Arg Lys Pro Val
   225                 230                 235                 240
   gac aag ttc aaa gac tgc cat ctg gcc cgg gtc cct tct cat gcc gtt     768
   Asp Lys Phe Lys Asp Cys His Leu Ala Arg Val Pro Ser His Ala Val
                   245                 250                 255
   gtg gca cga agt gtg aat ggc aag gag gat gcc atc tgg aat ctt ctc     816
   Val Ala Arg Ser Val Asn Gly Lys Glu Asp Ala Ile Trp Asn Leu Leu
               260                 265                 270
   cgc cag gca cag gaa aag ttt gga aag gac aag tca ccg aaa ttc cag     864
   Arg Gln Ala Gln Glu Lys Phe Gly Lys Asp Lys Ser Pro Lys Phe Gln
           275                 280                 285
   ctc ttt ggc tcc cct agt ggg cag aaa gat ctg ctg ttc aag gac tct      912
   Leu Phe Gly Ser Pro Ser Gly Gln Lys Asp Leu Leu Phe Lys Asp Ser
       290                 295                 300
   gcc att ggg ttt tcg agg gtg ccc ccg agg ata gat tct ggg ctg tac      960
   Ala Ile Gly Phe Ser Arg Val Pro Pro Arg Ile Asp Ser Gly Leu Tyr
   305                 310                 315                 320
   ctt ggc tcc ggc tac ttc act gcc atc cag aac ttg agg aaa agt gag     1008
   Leu Gly Ser Gly Tyr Phe Thr Ala Ile Gln Asn Leu Arg Lys Ser Glu
                   325                 330                 335
   gag gaa gtg gct gcc cgg cgt gcg cgg gtc gtg tgg tgt gcg gtg ggc     1056
   Glu Glu Val Ala Ala Arg Arg Ala Arg Val Val Trp Cys Ala Val Gly
               340                 345                 350
   gag cag gag ctg cgc aag tgt aac cag tgg agt ggc ttg agc gaa ggc     1104
   Glu Gln Glu Leu Arg Lys Cys Asn Gln Trp Ser Gly Leu Ser Glu Gly
           355                 360                 365
   agc gtg acc tgc tcc tcg gcc tcc acc aca gag gac tgc atc gcc ctg     1152
   Ser Val Thr Cys Ser Ser Ala Ser Thr Thr Glu Asp Cys Ile Ala Leu
       370                 375                 380
   gtg ctg aaa gga gaa gct gat gcc atg agt ttg gat gaa gga tat gtg     1200
   Val Leu Lys Gly Glu Ala Asp Ala Met Ser Leu Asp Glu Gly Tyr Val
   385                 390                 395                 400
   tac act gca ggc aaa tgt ggt ttg gtg cct gtc ctg gca gag aac tac     1248
   Tyr Thr Ala Gly Lys Cys Gly Leu Val Pro Val Leu Ala Glu Asn Tyr
                   405                 410                 415
   aaa tcc caa caa agc agt gac cct gat cct aac tgt gtg gat aga cct     1296
   Lys Ser Gln Gln Ser Ser Asp Pro Asp Pro Asn Cys Val Asp Arg Pro
               420                 425                 430
   gtg gaa gga tat ctt gct gtg gcg gtg gtt agg aga tca gac act agc     1344
   Val Glu Gly Tyr Leu Ala Val Ala Val Val Arg Arg Ser Asp Thr Ser
           435                 440                 445
   ctt acc tgg aac tct gtg aaa ggc aag aag tcc tgc cac acc gcc gtg     1392
   Leu Thr Trp Asn Ser Val Lys Gly Lys Lys Ser Cys His Thr Ala Val
       450                 455                 460
   gac agg act gca ggc tgg aat atc ccc atg ggc ctg ctc ttc aac cag     1440
   Asp Arg Thr Ala Gly Trp Asn Ile Pro Met Gly Leu Leu Phe Asn Gln
   465                 470                 475                 480
   acg ggc tcc tgc aaa ttt gat gaa tat ttc agt caa agc tgt gcc cct     1488
   Thr Gly Ser Cys Lys Phe Asp Glu Tyr Phe Ser Gln Ser Cys Ala Pro
                   485                 490                 495
   ggg tct gac ccg aga tct aat ctc tgt gct ctg tgt aat ggc gac gag     1536
   Gly Ser Asp Pro Arg Ser Asn Leu Cys Ala Leu Cys Asn Gly Asp Glu
               500                 505                 510
   cag ggt gag aat aag tgc gtg ccc aac agc aac gag aga tac tac ggc     1584
   Gln Gly Glu Asn Lys Cys Val Pro Asn Ser Asn Glu Arg Tyr Tyr Gly
           515                 520                 525
   tac act ggg gct ttc cgg tgc ctg gct gag aat gct gga gac gtt gca     1632
   Tyr Thr Gly Ala Phe Arg Cys Leu Ala Glu Asn Ala Gly Asp Val Ala
       530                 535                 540
   ttt gtg aaa gat gtc act gtc ttg cag aac act gat gga aat aac aat     1680
   Phe Val Lys Asp Val Thr Val Leu Gln Asn Thr Asp Gly Asn Asn Asn
   545                 550                 555                 560
   gag gca tgg gct aag gat ttg aag ctg gca gac ttt gcg ctg ctg tgc      1728
   Glu Ala Trp Ala Lys Asp Leu Lys Leu Ala Asp Phe Ala Leu Leu Cys
                   565                 570                 575
   ctc gat ggc aaa cgg aag cct gtg act gag gct aga agc tgc cat ctt      1776
   Leu Asp Gly Lys Arg Lys Pro Val Thr Glu Ala Arg Ser Cys His Leu
           580                 585                 590
   gcc atg gcc ccg aat cat gcc gtg gtg tct cgg atg gat aag gtg gaa      1824
   Ala Met Ala Pro Asn His Ala Val Val Ser Arg Met Asp Lys Val Glu
       595                 600                 605
   cgc ctg aaa cag gtg ttg ctc cac caa cag gct aaa ttt ggg aga aat      1872
   Arg Leu Lys Gln Val Leu Leu His Gln Gln Ala Lys Phe Gly Arg Asn
       610                 615                 620
   gga tct gac tgc ccg gac aag ttt tgc tta ttc cag tct gaa acc aaa      1920
   Gly Ser Asp Cys Pro Asp Lys Phe Cys Leu Phe Gln Ser Glu Thr Lys
   625                 630                 635                 640
   aac ctt ctg ttc aat gac aac act gag tgt ctg gcc aga ctc cat gga      1968
   Asn Leu Leu Phe Asn Asp Asn Thr Glu Cys Leu Ala Arg Leu His Gly
                   645                 650                 655
   aaa aca aca tat gaa aaa tat ttg gga cca cag tat gtc gca ggc att      2016
   Lys Thr Thr Tyr Glu Lys Tyr Leu Gly Pro Gln Tyr Val Ala Gly Ile
               660                 665                 670
   act aat ctg aaa aag tgc tca acc tcc ccc ctc ctg gaa gcc tgt gaa      2064
   Thr Asn Leu Lys Lys Cys Ser Thr Ser Pro Leu Leu Glu Ala Cys Glu
           675                 680                 685
   ttc ctc agg aag taa                                                  2079
   Phe Leu Arg Lys
       690
<210> 14
   <211> 692
   <212> PRT
   <213> Homo sapiens
<400> 14
   Gly Arg Arg Arg Arg Ser Val Gln Trp Cys Ala Val Ser Gln Pro Glu
   1               5                   10                  15
   Ala Thr Lys Cys Phe Gln Trp Gln Arg Asn Met Arg Lys Val Arg Gly
               20                  25                  30
   Pro Pro Val Ser Cys Ile Lys Arg Asp Ser Pro Ile Gln Cys Ile Gln
           35                  40                  45
   Ala Ile Ala Glu Asn Arg Ala Asp Ala Val Thr Leu Asp Gly Gly Phe
       50                  55                  60
   Ile Tyr Glu Ala Gly Leu Ala Pro Tyr Lys Leu Arg Pro Val Ala Ala
   65                  70                  75                  80
   Glu Val Tyr Gly Thr Glu Arg Gln Pro Arg Thr His Tyr Tyr Ala Val
                   85                  90                  95
   Ala Val Val Asn Lys Gly Gly Ser Phe Gln Leu Asn Glu Leu Gln Gly
   100 105 110
   Leu Lys Ser Cys His Thr Gly Leu Arg Arg Thr Ala Gly Trp Asn Val
   115 120 125
   Pro Ile Gly Thr Leu Arg Pro Phe Leu Asn Trp Thr Gly Pro Pro Glu
   130 135 140
   Pro Ile Glu Ala Ala Val Ala Arg Phe Phe Ser Ala Ser Cys Val Pro
   145 150 155 160
   Gly Ala Asp Lys Gly Gln Phe Pro Asn Leu Cys Arg Leu Cys Ala Gly
   165 170 175
   Thr Gly Glu Asn Lys Cys Ala Phe Ser Ser Gln Glu Pro Tyr Phe Ser
   180 185 190
   Tyr Ser Gly Ala Phe Lys Cys Leu Arg Asp Gly Ala Gly Asp Val Ala
   195 200 205
   Phe Ile Arg Glu Ser Thr Val Phe Glu Asp Leu Ser Asp Glu Ala Glu
   210 215 220
   Arg Asp Glu Tyr Glu Leu Leu Cys Pro Asp Asn Thr Arg Lys Pro Val
   225 230 235 240
   Asp Lys Phe Lys Asp Cys His Leu Ala Arg Val Pro Ser His Ala Val
   245 250 255
   Val Ala Arg Ser Val Asn Gly Lys Glu Asp Ala Ile Trp Asn Leu Leu
   260 265 270
   Arg Gln Ala Gln Glu Lys Phe Gly Lys Asp Lys Ser Pro Lys Phe Gln
   275 280 285
   Leu Phe Gly Ser Pro Ser Gly Gln Lys Asp Leu Leu Phe Lys Asp Ser
   290 295 300
   Ala Ile Gly Phe Ser Arg Val Pro Pro Arg Ile Asp Ser Gly Leu Tyr
   305 310 315 320
   Leu Gly Ser Gly Tyr Phe Thr Ala Ile Gln Asn Leu Arg Lys Ser Glu
   325 330 335
   Glu Glu Val Ala Ala Arg Arg Ala Arg Val Val Trp Cys Ala Val Gly
   340 345 350
   Glu Gln Glu Leu Arg Lys Cys Asn Gln Trp Ser Gly Leu Ser Glu Gly
   355 360 365
   Ser Val Thr Cys Ser Ser Ala Ser Thr Thr Glu Asp Cys Ile Ala Leu
   370 375 380
   Val Leu Lys Gly Glu Ala Asp Ala Met Ser Leu Asp Glu Gly Tyr Val
   385 390 395 400
   Tyr Thr Ala Gly Lys Cys Gly Leu Val Pro Val Leu Ala Glu Asn Tyr
   405 410 415
   Lys Ser Gln Gln Ser Ser Asp Pro Asp Pro Asn Cys Val Asp Arg Pro
   420 425 430
   Val Glu Gly Tyr Leu Ala Val Ala Val Val Arg Arg Ser Asp Thr Ser
   435 440 445
   Leu Thr Trp Asn Ser Val Lys Gly Lys Lys Ser Cys His Thr Ala Val
   450 455 460
   Asp Arg Thr Ala Gly Trp Asn Ile Pro Met Gly Leu Leu Phe Asn Gln
   465 470 475 480
   Thr Gly Ser Cys Lys Phe Asp Glu Tyr Phe Ser Gln Ser Cys Ala Pro
   485 490 495
   Gly Ser Asp Pro Arg Ser Asn Leu Cys Ala Leu Cys Asn Gly Asp Glu
   500 505 510
   Gln Gly Glu Asn Lys Cys Val Pro Asn Ser Asn Glu Arg Tyr Tyr Gly
   515 520 525
   Tyr Thr Gly Ala Phe Arg Cys Leu Ala Glu Asn Ala Gly Asp Val Ala
   530 535 540
   Phe Val Lys Asp Val Thr Val Leu Gln Asn Thr Asp Gly Asn Asn Asn
   545 550 555 560
   Glu Ala Trp Ala Lys Asp Leu Lys Leu Ala Asp Phe Ala Leu Leu Cys
   565 570 575
   Leu Asp Gly Lys Arg Lys Pro Val Thr Glu Ala Arg Ser Cys His Leu
   580 585 590
   Ala Met Ala Pro Asn His Ala Val Val Ser Arg Met Asp Lys Val Glu
   595 600 605
   Arg Leu Lys Gln Val Leu Leu His Gln Gln Ala Lys Phe Gly Arg Asn
   610 615 620
   Gly Ser Asp Cys Pro Asp Lys Phe Cys Leu Phe Gln Ser Glu Thr Lys
   625 630 635 640
   Asn Leu Leu Phe Asn Asp Asn Thr Glu Cys Leu Ala Arg Leu His Gly
   645 650 655
   Lys Thr Thr Tyr Glu Lys Tyr Leu Gly Pro Gln Tyr Val Ala Gly Ile
   660 665 670
   Thr Asn Leu Lys Lys Cys Ser Thr Ser Pro Leu Leu Glu Ala Cys Glu
   675 680 685
   Phe Leu Arg Lys
   690
<210> 15
   <211> 692
   <212> PRT
   <213> Homo sapiens
<400> 15
   Gly Arg Arg Arg Arg Ser Val Gln Trp Cys Ala Val Ser Gln Pro Glu
   1               5                   10                  15
   Ala Thr Lys Cys Phe Gln Trp Gln Arg Asn Met Arg Lys Val Arg Gly
               20                  25                  30
   Pro Pro Val Ser Cys Ile Lys Arg Asp Ser Pro Ile Gln Cys Ile Gln
           35                  40                  45
   Ala Ile Ala Glu Asn Arg Ala Asp Ala Val Thr Leu Asp Gly Gly Phe
       50                  55                  60
   Ile Tyr Glu Ala Gly Leu Ala Pro Tyr Lys Leu Arg Pro Val Ala Ala
   65                  70                  75                  80
   Glu Val Tyr Gly Thr Glu Arg Gln Pro Arg Thr His Tyr Tyr Ala Val
                   85                  90                  95
   Ala Val Val Asn Lys Gly Gly Ser Phe Gln Leu Asn Glu Leu Gln Gly
               100                 105                 110
   Leu Lys Ser Cys His Thr Gly Leu Arg Arg Thr Ala Gly Trp Asn Val
           115                 120                 125
   Pro Ile Gly Thr Leu Arg Pro Phe Leu Asn Trp Thr Gly Pro Pro Glu
       130                 135                 140
   Pro Ile Glu Ala Ala Val Ala Arg Phe Phe Ser Ala Ser Cys Val Pro
   145                 150                 155                 160
   Gly Ala Asp Lys Gly Gln Phe Pro Asn Leu Cys Arg Leu Cys Ala Gly
                   165                 170                 175
   Thr Gly Glu Asn Lys Cys Ala Phe Ser Ser Gln Glu Pro Tyr Phe Ser
                   180                 185                 190
   Tyr Ser Gly Ala Phe Lys Cys Leu Arg Asp Gly Ala Gly Asp Val Ala
               195                 200                 205
   Phe Ile Arg Glu Ser Thr Val Phe Glu Asp Leu Ser Asp Glu Ala Glu
       210                 215                 220
   Arg Asp Glu Tyr Glu Leu Leu Cys Pro Asp Asn Thr Arg Lys Pro Val
   225                 230                 235                 240
   Asp Lys Phe Lys Asp Cys His Leu Ala Arg Val Pro Ser His Ala Val
                   245                 250                 255
   Val Ala Arg Ser Val Asn Gly Lys Glu Asp Ala Ile Trp Asn Leu Leu
               260                 265                 270
   Arg Gln Ala Gln Glu Lys Phe Gly Lys Asp Lys Ser Pro Lys Phe Gln
           275                 280                 285
   Leu Phe Gly Ser Pro Ser Gly Gln Lys Asp Leu Leu Phe Lys Asp Ser
       290                 295                 300
   Ala Ile Gly Phe Ser Arg Val Pro Pro Arg Ile Asp Ser Gly Leu Tyr
   305                 310                 315                 320
   Leu Gly Ser Gly Tyr Phe Thr Ala Ile Gln Asn Leu Arg Lys Ser Glu
                   325                 330                 335
   Glu Glu Val Ala Ala Arg Arg Ala Arg Val Val Trp Cys Ala Val Gly
               340                 345                 350
   Glu Gln Glu Leu Arg Lys Cys Asn Gln Trp Ser Gly Leu Ser Glu Gly
           355                 360                 365
   Ser Val Thr Cys Ser Ser Ala Ser Thr Thr Glu Asp Cys Ile Ala Leu
       370                 375                 380
   Val Leu Lys Gly Glu Ala Asp Ala Met Ser Leu Asp Glu Gly Tyr Val
   385                 390                 395                 400
   Tyr Thr Ala Gly Lys Cys Gly Leu Val Pro Val Leu Ala Glu Asn Tyr
                   405                 410                 415
   Lys Ser Gln Gln Ser Ser Asp Pro Asp Pro Asn Cys Val Asp Arg Pro
               420                 425                 430
   Val Glu Gly Tyr Leu Ala Val Ala Val Val Arg Arg Ser Asp Thr Ser
           435                 440                 445
   Leu Thr Trp Asn Ser Val Lys Gly Lys Lys Ser Cys His Thr Ala Val
   450 455 460
   Asp Arg Thr Ala Gly Trp Asn Ile Pro Met Gly Leu Leu Phe Asn Gln
   465 470 475 480
   Thr Gly Ser Cys Lys Phe Asp Glu Tyr Phe Ser Gln Ser Cys Ala Pro
   485 490 495
   Gly Ser Asp Pro Arg Ser Asn Leu Cys Ala Leu Cys Asn Gly Asp Glu
   500 505 510
   Gln Gly Glu Asn Lys Cys Val Pro Asn Ser Asn Glu Arg Tyr Tyr Gly
   515 520 525
   Tyr Thr Gly Ala Phe Arg Cys Leu Ala Glu Asn Ala Gly Asp Val Ala
   530 535 540
   Phe Val Lys Asp Val Thr Val Leu Gln Asn Thr Asp Gly Asn Asn Asn
   545 550 555 560
   Glu Ala Trp Ala Lys Asp Leu Lys Leu Ala Asp Phe Ala Leu Leu Cys
   565 570 575
   Leu Asp Gly Lys Arg Lys Pro Val Thr Glu Ala Arg Ser Cys His Leu
   580 585 590
   Ala Met Ala Pro Asn His Ala Val Val Ser Arg Met Asp Lys Val Glu
   595 600 605
   Arg Leu Lys Gln Val Leu Leu His Gln Gln Ala Lys Phe Gly Arg Asn
   610 615 620
   Gly Ser Asp Cys Pro Asp Lys Phe Cys Leu Phe Gln Ser Glu Thr Lys
   625 630 635 640
   Asn Leu Leu Phe Asn Asp Asn Thr Glu Cys Leu Ala Arg Leu His Gly
   645 650 655
   Lys Thr Thr Tyr Glu Lys Tyr Leu Gly Pro Gln Tyr Val Ala Gly Ile
   660 665 670
   Thr Asn Leu Lys Lys Cys Ser Thr Ser Pro Leu Leu Glu Ala Cys Glu
   675 680 685
   Phe Leu Arg Lys
   690
<210> 16
   <211> 2079
   <212> DNA
   <213> Artificial
<220>
   <223> Synthetic codon optimized human lactoferrin gene sequence
<400> 16
   gggcggcggc ggcgctcggt gcagtggtgc gccgtgtccc agcccgaggc gaccaagtgc 60
   ttccagtggc agcgcaacat gcggaaggtg cgcggcccgc cggtcagctg catcaagcgg 120
   gactccccca tccaatgcat ccaggccatc gcggagaacc gcgccgacgc ggtcaccctg 180
   gacggcgggt tcatctacga ggcggggctc gccccgtaca agctccgccc ggtggcggcg 240
   gaggtgtacg gcaccgagcg ccagccgcgc acgcactact acgcggtggc cgtcgtcaag 300
   aagggcgggt ccttccagct caacgagctg cagggcctga agtcgtgcca cacgggcctc 360
   cggcggacgg cgggctggaa cgtgcccatc ggcaccctgc gccccttcct gaactggacc 420
   ggcccgccgg agccgatcga ggccgccgtg gcccgcttct tcagcgcctc ctgcgtcccc 480
   ggcgccgaca agggccagtt cccgaacctc tgccggctct gcgccgggac gggcgagaac 540
   aagtgcgcct tctcctcgca ggagccgtac ttctcctact cgggcgcgtt caagtgcctc 600
   cgcgacgggg ccggcgacgt ggcgttcatc cgcgagtcca ccgtgttcga ggacctctcc 660
   gacgaggcgg agcgggacga gtacgagctg ctgtgccccg acaacacccg caagccggtg 720
   gacaagttca aggactgcca cctggcgcgg gtgccctcgc acgcggtcgt cgcccgcagc 780
   gtcaacggca aggaggacgc gatctggaac ctcctccgcc aggcccagga gaagttcggc 840
   aaggacaagt cccccaagtt ccagctcttc gggagcccca gcggccagaa ggacctcctc 900
   ttcaaggact ccgcgatcgg cttctcccgc gtccccccgc gcatcgactc cggcctgtac 960
   ctcggctccg ggtacttcac cgcgatccag aacctccgga agagcgagga ggaggtggcg 1020
   gcgcggcggg cccgcgtcgt gtggtgcgcc gtgggcgagc aggagctgcg gaagtgcaac 1080
   cagtggagcg gcctgagcga ggggtcggtg acctgctcgt ccgccagcac caccgaggac 1140
   tgcatcgcgc tcgtcctcaa gggggaggcc gacgcgatga gcctcgacgg ggggtacgtc 1200
   tacaccgccg gcaagtgcgg cctggtcccg gtcctggcgg agaactacaa gtcgcagcag 1260
   tccagcgacc ccgacccgaa ctgcgtggac cgccccgtcg agggctacct cgccgtggcc 1320
   gtcgtgcgcc ggtccgacac ctccctgacg tggaacagcg tcaagggcaa gaagagctgc 1380
   cacaccgccg tggaccgcac cgccggctgg aacatcccga tgggcctcct cttcaaccag 1440
   accggctcct gcaagttcga cgagtacttc tcccagtcct gcgcccccgg ctcggacccc 1500
   cgctccaacc tgtgcgccct ctgcatcggg gacgagcagg gcgagaacaa gtgcgtgccc 1560
   aacagcaacg agcggtacta cggctacacg ggggccttcc gctgcctggc ggagaacgcc 1620
   ggggacgtcg cgttcgtgaa ggacgtgacc gtgctgcaaa acacggacgg gaacaacaac 1680
   gaggcgtggg cgaaggacct caagctcgcc gacttcgccc tgctgtgcct cgacggcaag 1740
   cgcaagcccg tcaccgaggc gcggtcctgc cacctggcga tggcccccaa ccacgccgtc 1800
   gtctcccgca tggacaaggt cgagcgcctc aagcaggtgc tcctgcacca gcaggccaag 1860
   ttcggccgga acggcagcga ctgcccggac aagttctgcc tgttccagtc ggagaccaag 1920
   aacctcctct tcaacgacaa caccgagtgc ctggcgcgcc tccacggcaa gaccacctac 1980
   gagaagtacc tcggcccgca gtacgtcgcc ggcatcacca acctcaagaa gtgctccacc 2040
   tcccccctcc tggaggcgtg cgagttcctc cgcaagtga 2079
<210> 17
   <211> 1830
   <212> DNA
   <213> Artificial
<220>
   <223> Synthetic fusion Gt1 SP/optimized HSA sequence
<220>
   <221> CDS
   <222> (1)..(1827)
<400> 17
   atg gca tcc ata aat cgc ccc ata gtt ttc ttc aca gtt tgc ttg ttc       48
   Met Ala Ser Ile Asn Arg Pro Ile Val Phe Phe Thr Val Cys Leu Phe
   1               5                   10                  15
   ctc ttg tgc gat ggc tcc cta gcc gac gcc cac aag agc gag gtg gcc       96
   Leu Leu Cys Asp Gly Ser Leu Ala Asp Ala His Lys Ser Glu Val Ala
               20                  25                  30
   cac cgc ttc aag gac ctc ggc gag gag aac ttc aag gcc ctc gtg ctc      144
   His Arg Phe Lys Asp Leu Gly Glu Glu Asn Phe Lys Ala Leu Val Leu
           35                  40                  45
   atc gcc ttc gcc cag tac ctc cag cag tgc ccg ttc gag gac cac gtg      192
   Ile Ala Phe Ala Gln Tyr Leu Gln Gln Cys Pro Phe Glu Asp His Val
       50                  55                  60
   aag ctc gtg aac gag gtg acc gag ttc gcc aag acc tgc gtg gcc gac      240
   Lys Leu Val Asn Glu Val Thr Glu Phe Ala Lys Thr Cys Val Ala Asp
   65                  70                  75                  80
   gag agc gcc gag aac tgc gac aag agc ctc cac acc ctc ttc ggc gac      288
   Glu Ser Ala Glu Asn Cys Asp Lys Ser Leu His Thr Leu Phe Gly Asp
                   85                  90                  95
   aag ctc tgc acc gtg gcc acc ctc cgc gag acc tac ggc gag atg gcc      336
   Lys Leu Cys Thr Val Ala Thr Leu Arg Glu Thr Tyr Gly Glu Met Ala
               100                 105                 110
   gac tgc tgc gcc aag cag gag ccg gag cgc aac gag tgc ttc ctc gag      384
   Asp Cys Cys Ala Lys Gln Glu Pro Glu Arg Asn Glu Cys Phe Leu Glu
           115                 120                 125
   cac aag gac gac acc ccg aac ctc ccg cgc ctc gtg cgc ccg gag gtg      432
   His Lys Asp Asp Thr Pro Asn Leu Pro Arg Leu Val Arg Pro Glu Val
       130                 135                 140
   gac gtg atg tgc acc gcc ttc cac gac aac gag gag acc ttc ctc aag      480
   Asp Val Met Cys Thr Ala Phe His Asp Asn Glu Glu Thr Phe Leu Lys
   145                 150                 155                 160
   aag tac ctc tac gag atc gcc cgc cgc cac ccg tac ttc tac gcc ccg      528
   Lys Tyr Leu Tyr Glu Ile Ala Arg Arg His Pro Tyr Phe Tyr Ala Pro
                   165                 170                 175
   gag ctc ctc ttc ttc gcc aag cgc tac aag gcc gcc ttc acc gag tgc      576
   Glu Leu Leu Phe Phe Ala Lys Arg Tyr Lys Ala Ala Phe Thr Glu Cys
               180                 185                 190
   tgc cag gcc gcc gac aag gcc gcc tgc ctc ctc ccg aag ctc gac gag      624
   Cys Gln Ala Ala Asp Lys Ala Ala Cys Leu Leu Pro Lys Leu Asp Glu
           195                 200                 205
   ctc cgc gac gag ggc aag gcc tcc agc gcc aag cag cgc ctc aag tgc      672
   Leu Arg Asp Glu Gly Lys Ala Ser Ser Ala Lys Gln Arg Leu Lys Cys
       210                 215                 220
   gcc agc ctc cag aag ttc ggc gag cgc gcc ttc aag gcc tgg gcc gtg      720
   Ala Ser Leu Gln Lys Phe Gly Glu Arg Ala Phe Lys Ala Trp Ala Val
   225                 230                 235                 240
   gcc cgc ctc agc cag cgc ttc ccg aag gcc gag ttc gcc gag gtg tcc      768
   Ala Arg Leu Ser Gln Arg Phe Pro Lys Ala Glu Phe Ala Glu Val Ser
                   245                 250                 255
   aag ctc gtg acc gac ctc acc aag gtg cac acc gag tgc tgc cac ggc      816
   Lys Leu Val Thr Asp Leu Thr Lys Val His Thr Glu Cys Cys His Gly
               260                 265                 270
   gac ctc ctg gag tgc gcc gac gac cgc gcc gac ctc gcc aag tac atc      864
   Asp Leu Leu Glu Cys Ala Asp Asp Arg Ala Asp Leu Ala Lys Tyr Ile
           275                 280                 285
   tgc gag aac cag gac agc atc tcc agc aag ctc aag gag tgc tgc gag      912
   Cys Glu Asn Gln Asp Ser Ile Ser Ser Lys Leu Lys Glu Cys Cys Glu
       290                 295                 300
   aag ccg ctc ctg gag aag tcc cac tgc atc gcc gag gtg gag aac gac      960
   Lys Pro Leu Leu Glu Lys Ser His Cys Ile Ala Glu Val Glu Asn Asp
   305                 310                 315                 320
   gag atg ccg gcc gac ctc ccg tcc ctc gcc gcc gac ttc gtg gag agc     1008
   Glu Met Pro Ala Asp Leu Pro Ser Leu Ala Ala Asp Phe Val Glu Ser
                   325                 330                 335
   aag gac gtg tgc aag aac tac gcc gag gcc aag gac gtc ttc ctc ggc     1056
   Lys Asp Val Cys Lys Asn Tyr Ala Glu Ala Lys Asp Val Phe Leu Gly
               340                 345                 350
   atg ttc ctc tac gag tac gcc cgc cgc cac ccg gac tac tcc gtg gtg     1104
   Met Phe Leu Tyr Glu Tyr Ala Arg Arg His Pro Asp Tyr Ser Val Val
           355                 360                 365
   ctc ctc ctc cgc ctc gcc aag acc tac gag acc acc ctg gag aag tgc     1152
   Leu Leu Leu Arg Leu Ala Lys Thr Tyr Glu Thr Thr Leu Glu Lys Cys
       370                 375                 380
   tgc gcc gcc gcc gac ccg cac gag tgc tac gcc aag gtg ttc gac gag     1200
   Cys Ala Ala Ala Asp Pro His Glu Cys Tyr Ala Lys Val Phe Asp Glu
   385                 390                 395                 400
   ttc aag ccg ctc gtg gag gag ccg cag aac ctc atc aag cag aac tgc     1248
   Phe Lys Pro Leu Val Glu Glu Pro Gln Asn Leu Ile Lys Gln Asn Cys
                   405                 410                 415
   gag ctc ttc gag cag ctc ggc gag tac aag ttc cag aac gcc ctc ctc     1296
   Glu Leu Phe Glu Gln Leu Gly Glu Tyr Lys Phe Gln Asn Ala Leu Leu
               420                 425                 430
   gtg cgc tac acc aag aag gtg ccg cag gtg tcc acc ccg acc ctc gtg     1344
   Val Arg Tyr Thr Lys Lys Val Pro Gln Val Ser Thr Pro Thr Leu Val
           435                 440                 445
   gag gtg tcc cgc aac ctc ggc aag gtg ggc agc aag tgc tgc aag cac     1392
   Glu Val Ser Arg Asn Leu Gly Lys Val Gly Ser Lys Cys Cys Lys His
       450                 455                 460
   ccg gag gcc aag cgc atg ccg tgc gcc gag gac tac ctc tcc gtg gtg      1440
   Pro Glu Ala Lys Arg Met Pro Cys Ala Glu Asp Tyr Leu Ser Val Val
   465                 470                 475                 480
   ctc aac cag ctc tgc gtg ctc cac gag aag acc ccg gtg agc gac cgc      1488
   Leu Asn Gln Leu Cys Val Leu His Glu Lys Thr Pro Val Ser Asp Arg
                   485                 490                 495
   gtg acc aag tgc tgc acc gag agc ctc gtg aac cgc cgc ccg tgc ttc      1536
   Val Thr Lys Cys Cys Thr Glu Ser Leu Val Asn Arg Arg Pro Cys Phe
               500                 505                 510
   tcc gcc ctg gag gtc gac gag acc tac gtc ccg aag gag ttc aac gcc      1584
   Ser Ala Leu Glu Val Asp Glu Thr Tyr Val Pro Lys Glu Phe Asn Ala
           515                 520                 525
   gag acc ttc acc ttc cac gcc gac atc tgc acc ctc tcc gag aag gag      1632
   Glu Thr Phe Thr Phe His Ala Asp Ile Cys Thr Leu Ser Glu Lys Glu
       530                 535                 540
   cgc cag atc aag aag cag acc gcc ctc gtc gag ctc gtg aag cac aag      1680
   Arg Gln Ile Lys Lys Gln Thr Ala Leu Val Glu Leu Val Lys His Lys
   545                 550                 555                 560
   ccg aag gcc acc aag gag cag ctc aag gcc gtg atg gac gac ttc gcc      1728
   Pro Lys Ala Thr Lys Glu Gln Leu Lys Ala Val Met Asp Asp Phe Ala
                   565                 570                 575
   gcc ttc gtg gag aag tgc tgc aag gcc gac gac aag gag acc tgc ttc      1776
   Ala Phe Val Glu Lys Cys Cys Lys Ala Asp Asp Lys Glu Thr Cys Phe
               580                 585                 590
   gcc gag gag ggc aag aag ctc gtg gcc gcc agc cag gcc gcc ctc ggc      1824
   Ala Glu Glu Gly Lys Lys Leu Val Ala Ala Ser Gln Ala Ala Leu Gly
           595                 600                 605
   ctc tga                                                              1830
   Leu
<210> 18
   <211> 609
   <212> PRT
   <213> Artificial
<220>
   <223> Synthetic Construct
<400> 18
   Met Ala Ser Ile Asn Arg Pro Ile Val Phe Phe Thr Val Cys Leu Phe
   1               5                   10                  15
   Leu Leu Cys Asp Gly Ser Leu Ala Asp Ala His Lys Ser Glu Val Ala
               20                  25                  30
   His Arg Phe Lys Asp Leu Gly Glu Glu Asn Phe Lys Ala Leu Val Leu
           35                  40                  45
   Ile Ala Phe Ala Gln Tyr Leu Gln Gln Cys Pro Phe Glu Asp His Val
       50                  55                  60
   Lys Leu Val Asn Glu Val Thr Glu Phe Ala Lys Thr Cys Val Ala Asp
   65                  70                  75                  80
   Glu Ser Ala Glu Asn Cys Asp Lys Ser Leu His Thr Leu Phe Gly Asp
                   85                  90                  95
   Lys Leu Cys Thr Val Ala Thr Leu Arg Glu Thr Tyr Gly Glu Met Ala
               100                 105                 110
   Asp Cys Cys Ala Lys Gln Glu Pro Glu Arg Asn Glu Cys Phe Leu Glu
           115                 120                 125
   His Lys Asp Asp Thr Pro Asn Leu Pro Arg Leu Val Arg Pro Glu Val
       130                 135                 140
   Asp Val Met Cys Thr Ala Phe His Asp Asn Glu Glu Thr Phe Leu Lys
   145                 150                 155                 160
   Lys Tyr Leu Tyr Glu Ile Ala Arg Arg His Pro Tyr Phe Tyr Ala Pro
                   165                 170                 175
   Glu Leu Leu Phe Phe Ala Lys Arg Tyr Lys Ala Ala Phe Thr Glu Cys
               180                 185                 190
   Cys Gln Ala Ala Asp Lys Ala Ala Cys Leu Leu Pro Lys Leu Asp Glu
           195                 200                 205
   Leu Arg Asp Glu Gly Lys Ala Ser Ser Ala Lys Gln Arg Leu Lys Cys
       210                 215                 220
   Ala Ser Leu Gln Lys Phe Gly Glu Arg Ala Phe Lys Ala Trp Ala Val
   225                 230                 235                 240
   Ala Arg Leu Ser Gln Arg Phe Pro Lys Ala Glu Phe Ala Glu Val Ser
                   245                 250                 255
   Lys Leu Val Thr Asp Leu Thr Lys Val His Thr Glu Cys Cys His Gly
               260                 265                 270
   Asp Leu Leu Glu Cys Ala Asp Asp Arg Ala Asp Leu Ala Lys Tyr Ile
           275                 280                 285
   Cys Glu Asn Gln Asp Ser Ile Ser Ser Lys Leu Lys Glu Cys Cys Glu
       290                 295                 300
   Lys Pro Leu Leu Glu Lys Ser His Cys Ile Ala Glu Val Glu Asn Asp
   305                 310                 315                 320
   Glu Met Pro Ala Asp Leu Pro Ser Leu Ala Ala Asp Phe Val Glu Ser
                   325                 330                 335
   Lys Asp Val Cys Lys Asn Tyr Ala Glu Ala Lys Asp Val Phe Leu Gly
   340 345 350
   Met Phe Leu Tyr Glu Tyr Ala Arg Arg His Pro Asp Tyr Ser Val Val
   355 360 365
   Leu Leu Leu Arg Leu Ala Lys Thr Tyr Glu Thr Thr Leu Glu Lys Cys
   370 375 380
   Cys Ala Ala Ala Asp Pro His Glu Cys Tyr Ala Lys Val Phe Asp Glu
   385 390 395 400
   Phe Lys Pro Leu Val Glu Glu Pro Gln Asn Leu Ile Lys Gln Asn Cys
   405 410 415
   Glu Leu Phe Glu Gln Leu Gly Glu Tyr Lys Phe Gln Asn Ala Leu Leu
   420 425 430
   Val Arg Tyr Thr Lys Lys Val Pro Gln Val Ser Thr Pro Thr Leu Val
   435 440 445
   Glu Val Ser Arg Asn Leu Gly Lys Val Gly Ser Lys Cys Cys Lys His
   450 455 460
   Pro Glu Ala Lys Arg Met Pro Cys Ala Glu Asp Tyr Leu Ser Val Val
   465 470 475 480
   Leu Asn Gln Leu Cys Val Leu His Glu Lys Thr Pro Val Ser Asp Arg
   485 490 495
   Val Thr Lys Cys Cys Thr Glu Ser Leu Val Asn Arg Arg Pro Cys Phe
   500 505 510
   Ser Ala Leu Glu Val Asp Glu Thr Tyr Val Pro Lys Glu Phe Asn Ala
   515 520 525
   Glu Thr Phe Thr Phe His Ala Asp Ile Cys Thr Leu Ser Glu Lys Glu
   530 535 540
   Arg Gln Ile Lys Lys Gln Thr Ala Leu Val Glu Leu Val Lys His Lys
   545 550 555 560
   Pro Lys Ala Thr Lys Glu Gln Leu Lys Ala Val Met Asp Asp Phe Ala
   565 570 575
   Ala Phe Val Glu Lys Cys Cys Lys Ala Asp Asp Lys Glu Thr Cys Phe
   580 585 590
   Ala Glu Glu Gly Lys Lys Leu Val Ala Ala Ser Gln Ala Ala Leu Gly
   595 600 605
   Leu
<210> 19
   <211> 609
   <212> PRT
   <213> Artificial
<220>
   <223> Synthetic fusion Gt1 SP/optimized HSA sequence
<400> 19
   Met Ala Ser Ile Asn Arg Pro Ile Val Phe Phe Thr Val Cys Leu Phe
   1               5                   10                  15
   Leu Leu Cys Asp Gly Ser Leu Ala Asp Ala His Lys Ser Glu Val Ala
               20                  25                  30
   His Arg Phe Lys Asp Leu Gly Glu Glu Asn Phe Lys Ala Leu Val Leu
           35                  40                  45
   Ile Ala Phe Ala Gln Tyr Leu Gln Gln Cys Pro Phe Glu Asp His Val
       50                  55                  60
   Lys Leu Val Asn Glu Val Thr Glu Phe Ala Lys Thr Cys Val Ala Asp
   65                  70                  75                  80
   Glu Ser Ala Glu Asn Cys Asp Lys Ser Leu His Thr Leu Phe Gly Asp
                   85                  90                  95
   Lys Leu Cys Thr Val Ala Thr Leu Arg Glu Thr Tyr Gly Glu Met Ala
               100                 105                 110
   Asp Cys Cys Ala Lys Gln Glu Pro Glu Arg Asn Glu Cys Phe Leu Glu
           115                 120                 125
   His Lys Asp Asp Thr Pro Asn Leu Pro Arg Leu Val Arg Pro Glu Val
       130                 135                 140
   Asp Val Met Cys Thr Ala Phe His Asp Asn Glu Glu Thr Phe Leu Lys
   145                 150                 155                 160
   Lys Tyr Leu Tyr Glu Ile Ala Arg Arg His Pro Tyr Phe Tyr Ala Pro
                   165                 170                 175
   Glu Leu Leu Phe Phe Ala Lys Arg Tyr Lys Ala Ala Phe Thr Glu Cys
               180                 185                 190
   Cys Gln Ala Ala Asp Lys Ala Ala Cys Leu Leu Pro Lys Leu Asp Glu
           195                 200                 205
   Leu Arg Asp Glu Gly Lys Ala Ser Ser Ala Lys Gln Arg Leu Lys Cys
       210                 215                 220
   Ala Ser Leu Gln Lys Phe Gly Glu Arg Ala Phe Lys Ala Trp Ala Val
   225 230 235 240
   Ala Arg Leu Ser Gln Arg Phe Pro Lys Ala Glu Phe Ala Glu Val Ser
   245 250 255
   Lys Leu Val Thr Asp Leu Thr Lys Val His Thr Glu Cys Cys His Gly
   260 265 270
   Asp Leu Leu Glu Cys Ala Asp Asp Arg Ala Asp Leu Ala Lys Tyr Ile
   275 280 285
   Cys Glu Asn Gln Asp Ser Ile Ser Ser Lys Leu Lys Glu Cys Cys Glu
   290 295 300
   Lys Pro Leu Leu Glu Lys Ser His Cys Ile Ala Glu Val Glu Asn Asp
   305 310 315 320
   Glu Met Pro Ala Asp Leu Pro Ser Leu Ala Ala Asp Phe Val Glu Ser
   325 330 335
   Lys Asp Val Cys Lys Asn Tyr Ala Glu Ala Lys Asp Val Phe Leu Gly
   340 345 350
   Met Phe Leu Tyr Glu Tyr Ala Arg Arg His Pro Asp Tyr Ser Val Val
   355 360 365
   Leu Leu Leu Arg Leu Ala Lys Thr Tyr Glu Thr Thr Leu Glu Lys Cys
   370 375 380
   Cys Ala Ala Ala Asp Pro His Glu Cys Tyr Ala Lys Val Phe Asp Glu
   385 390 395 400
   Phe Lys Pro Leu Val Glu Glu Pro Gln Asn Leu Ile Lys Gln Asn Cys
   405 410 415
   Glu Leu Phe Glu Gln Leu Gly Glu Tyr Lys Phe Gln Asn Ala Leu Leu
   420 425 430
   Val Arg Tyr Thr Lys Lys Val Pro Gln Val Ser Thr Pro Thr Leu Val
   435 440 445
   Glu Val Ser Arg Asn Leu Gly Lys Val Gly Ser Lys Cys Cys Lys His
   450 455 460
   Pro Glu Ala Lys Arg Met Pro Cys Ala Glu Asp Tyr Leu Ser Val Val
   465 470 475 480
   Leu Asn Gln Leu Cys Val Leu His Glu Lys Thr Pro Val Ser Asp Arg
   485 490 495
   Val Thr Lys Cys Cys Thr Glu Ser Leu Val Asn Arg Arg Pro Cys Phe
               500                 505                 510
   Ser Ala Leu Glu Val Asp Glu Thr Tyr Val Pro Lys Glu Phe Asn Ala
           515                 520                 525
   Glu Thr Phe Thr Phe His Ala Asp Ile Cys Thr Leu Ser Glu Lys Glu
       530                 535                 540
   Arg Gln Ile Lys Lys Gln Thr Ala Leu Val Glu Leu Val Lys His Lys
   545                 550                 555                 560
   Pro Lys Ala Thr Lys Glu Gln Leu Lys Ala Val Met Asp Asp Phe Ala
                   565                 570                 575
   Ala Phe Val Glu Lys Cys Cys Lys Ala Asp Asp Lys Glu Thr Cys Phe
               580                 585                 590
   Ala Glu Glu Gly Lys Lys Leu Val Ala Ala Ser Gln Ala Ala Leu Gly
           595                 600                 605
   Leu